# EUROPEAN PATENT SPECIFICATION

(11) **EP 1 047 431 B1**
(45) Date of publication and mention of the grant of the patent: **12.12.2012**
(21) Application number: 99904212.0
(22) Date of filing: 22.01.1999
(51) Int. Cl.: A61K 31/65, A61K 35/00

(54) **PHARMACEUTICALLY ACTIVE COMPOUNDS AND METHODS OF USE THEREOF**
PHARMAZEUTISCH AKTIVE VERBINDUNGEN UND VERFAHREN ZU DEREN VERWENDUNG
COMPOSES ACTIFS DU POINT DE VUE PHARMACEUTIQUE ET LEURS METHODES D'UTILISATION

(30) Priority: 23.01.1998 US 72262 P
(43) Date of publication of application: 02.11.2000
(73) Proprietor: TRUSTEES OF TUFTS COLLEGE, Medford, MA 02155 (US)
(72) Inventor: LEVY, Stuart, B., Boston, MA 02116 (US); NELSON, Mark, L., Wellesley, MA (US)
(74) Representative: Plougmann & Vingtoft A/S
(86) International application number: PCT/US1999/001393
(87) International publication number: WO 1999/037307

(56) References cited:
- WO-A-84/01895
- WO-A-93/08806
- WO-A-95/22529
- WO-A1-93/08806
- US-A- 3 901 942
- US-A- 3 901 942
- US-A- 4 806 529
- US-A- 5 064 821
- US-A- 5 258 372
- US-A- 5 589 470
- US-A- 5 811 412

## Description

The development of the tetracycline antibiotics was the direct result of a systematic screening of soil specimens collected from many parts of the world for evidence of microorganisms capable of producing bacteriocidal and/or bacteriostatic compositions. The first of these novel compounds was introduced in 1948 under the name chlortetracycline. Two years later oxytetracycline became available. The detailed elucidation of the chemical structure of these agents confirmed their similarity and furnished the analytical bases for the production of a third member of this group in 1952, tetracycline. By 1957, a new family of tetracycline compositions characterized chemically by the absence of the ring-attached CH₃ group present in the earlier compositions was prepared and became publicly available in 1967; and minocycline was in use by 1972. For clarity, for general ease of understanding, and for comparison purposes, these individual tetracycline-type agents are structurally compared within Table 1 below, with reference being made in that table to the following structural formula:

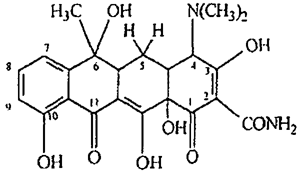

**Table 1**

| Congener | Substituent(s) | At Carbon Position Nos. |
|---|---|---|
| Chlortetracycline | -Cl | (7) |
| Oxytetracycline | -OH,-H | (5) |
| Demeclocycline | -OH,-H;-Cl | (6;7) |
| Methacycline | -OH,-H;=CH₂ | (5;6) |
| Doxycycline | -OH,-H;-CH₃,-H | (5;6) |
| Minocycline | -H,-H;-N(CH₃)₂ | (6;7) |

The terms "tetracycline" or "tetracycline-type" compound include tetracycline and other tetracycline family members such as the above chlortetracycline, oxytetracycline, demeclocycline, methacycline, doxycycline, minocycline, etc. as well as other tetracycline compounds having the above general fused ring structure whether now known or subsequently discovered or developed. Additionally, numbered tetracycline ring positions as referred to herein are the same as designated in the above structural formula.

More recent research efforts have focused on developing new tetracycline antibiotic compositions effective under varying therapeutic conditions and routes of administration; and for developing new tetracycline analogues which might prove to be equal or more effective then the originally introduced tetracycline families beginning in 1948. Representative of such developments include U.S. Patent Nos. 3,957,980; 3,674,859; 2,980,584; 2,990,331; 3,062,717; 3,557,280; 4,018,889; 4,024,272; 4,126,680; 3,454,697; and 3,165,531. It will be understood that these issued patents are merely representative of the range of diversity of investigations seeking tetracycline and tetracycline analogue compositions which are pharmacologically active.

Historically, soon after their initial development and introduction, the tetracyclines regardless of specific formulation or chemical structure were found to be highly effective pharmacologically against rickettsiae; a number of gram-positive and gram-negative bacteria; and the agents responsible for lymphogranuloma venereum, inclusion conjunctivitis, and psittacosis. Hence, tetracyclines became known as "broad spectrum" antibiotics. With the subsequent establishment of their *in vitro* antimicrobial activity, effectiveness in experimental infections, and pharmacological properties, the tetracyclines as a class rapidly became widely used for therapeutic purposes. However, this widespread use of tetracyclines for both major and minor illnesses and diseases led directly to the emergence of resistance to these antibiotics even among highly susceptible bacterial species both commensal and pathogenic - as for example pneumococci and *Salmonella.* The rise of tetracycline-resistant organisms has resulted in a general decline in use of tetracyclines and tetracycline analogue compositions as antibiotics of choice.

Tetracycline resistance is often regulated - that is, inducible by tetracycline. Investigations of active tetracycline efflux systems and the details of the active efflux mechanism of action have been well documented and include the following publications, each of which is expressly incorporated by reference herein: Chopra, et al., J. Antimictobiol. Chemotherapy 8:5-21 (1981); Levy and McMurry, Biochem. Biophys. Res. Comm. 56:1060-1068 (1974); Levy and McMurry, Nature, 275:90-92 (1978); McMurry and Levy, Antimicrobial Agents and Chemotherapy, 114:201-209 (1978); McMurry et al., Proc. Nat. Acad. Sci. U.S.A. 77:3974-3977 (1980); Ball, et al., Biochem. Biophys. Pes. Comm. 93:74-81 (1980); Curiale and Levy, J. Bact., 151:209-2115 (1982); Mendez, et al., Plasmid, 3:99-108 (1980); Curiale, et al.. J Bact. 157:211-217 (1984); and Levy, S.B., Journal of Antimicrobial Chemotherapy, 24:1-3 (1989).

In addition, a second mechanism of tetracycline resistance for cells is known and in effect. This resistance mechanism involves a cytoplasmic protein which protects the intracellular ribosomes from the inhibitory action of tetracyclines. This form of tetracycline resistance is described within Burdett, V., J. Bact, 165:564-569 (1986); and Levy, S.B., J. Antimicrob. Chem., 24:1-3 (1989).

WO 93/08806 discloses a method of overcoming tetracycline resistance through the use of a combination of tetracycline and a blocking agent, which blocking agent may include certain 13-(substituted mercapto) tetracycline derivatives which are unsubstituted in the 9-position.

WO 95/22529 discloses certain 6-deoxy-5-oxy tetracycline derivatives which are substituted in the 9-position with alkylamido or alkylsulfonamido.

WO 84/01895, US 5,258,372, and US 4,806,529 disclose a method of overcoming bacterial resistance to tetracycline-type antibiotics by inhibiting the plasmid-mediated active tetracycline efflux system of a tetracycline-resistant cell by treating the cell with a tetracycline efflux blocking agent, which may include certain known tetracycline antibiotics such as doxycycline and sancycline.

US 3,901,942 discloses a method for preparing tetracycline derivatives substituted in the 7-position, but unsubstituted in the 9-position, by first obtaining tetracycline derivatives substituted in the 7- and 9-positions, transforming substituent in the 7-position into the desired substituent, and then eliminating the substituent in the 9-position.

US 5,064,821 discloses a method of overcoming tetracycline resistance through the administration of a synergistic combination of certain 13-(substituted mercapto) tetracycline derivatives which are unsubstituted in the 9-position, and other tetracycline-type antibiotics.

US 5,589,470 discloses a method of overcoming tetracycline resistance through the administration of a combination of a blocking agent such as C5 ester derivatives or 13-(substituted mercapto) derivatives of tetracycline which are unsubstituted in the 9-position, and other tetracycline-type antibiotics, as a synergistic combination.

### Summary of the Invention

The present invention relates to novel substituted tetracycline-type compounds, and pharmaceutical compositions including such compounds. These compounds are useful for treatment against both tetracycline-sensitive and resistant microorganisms such as bacteria, fungi, rickettsia and the like. It has been found that compounds of the invention are highly active against both gram-positive as well as some gram-negative tetracycline-sensitive and tetracycline-resistant bacteria.

In a first embodiment, tetracycline-type compounds are provided that are substituted by other than hydroxy and hydrogen at the 5- and 9-ring positions. These compounds are generally referred to herein as 5,9-substituted tetracyclines or 5,9-substituted compounds. Suitable 5-position substituents include saturated and unsaturated aliphatic and aromatic ethers and esters. Suitable 9-position substituents include alkyl, alkenyl, and alkynyl groups; heteroalkyl, heteroalkylene, and heteroalkynyl groups; and carbocyclic aryl and heteroaromatic groups.

Thus, the 5,9-substituted tetracyclines compounds of the invention have the following Formula I: 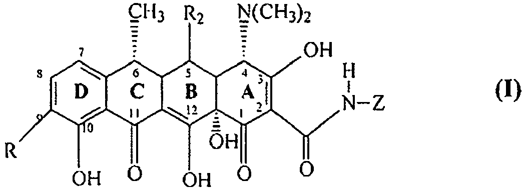
wherein R (9-position substituent) is alkyl preferably having from 1 to about 20 carbon atoms, more preferably 1 to about 12 carbon atoms; alkenyl preferably having from 2 to about 20 carbon atoms, more preferably 2 to about 12 carbon atoms; alkynyl preferably having from 2 to about 20 carbon atoms, more preferably 2 to about 12 carbon atoms; alkoxy preferably having from 1 to about 20 carbon atoms, more preferably 1 to about 12 carbon atoms; alkylthio preferably having from 1 to about 20 carbon atoms, more preferably 1 to about 12 carbon atoms; alkylsulfinyl preferably having from 1 to about 20 carbon atoms, more preferably 1 to about 12 carbon atoms; alkylsulfonyl preferably having from 1 to about 20 carbon atoms, more preferably 1 to about 12 carbon atoms; an alkyl group having an amino group attached thereto, preferably having from 1 to about 20 carbon atoms, more preferably 1 to about 12 carbon atoms; or an aryalkyl such as benzyl;
R² (5-position substituent) is alkanoyl preferably having from 1 to about 20 carbon atoms, more preferably 1 to about 12 carbon atoms; aroyl; alkaroyl; carbocyclic aryl; heteroaromatic; alkyl; alkenyl; alkynyl; alkoxy; alkylthio; alkylsulfinyl; alkylsulfonyl; an alkyl group having an amino group attached thereto; or an arylalkyl;
Z is hydrogen; alkyl; alkenyl; alkynyl; alkoxy; alkylthio; alkylsulfinyl; alkylsulfonyl; arylalkyl; carbocyclic aryl; an alkyl group having an amino group attached thereto; or heteroalicyclic; or a pharmaceutically acceptable salt thereof.
Z is hydrogen, a group as defined for R¹ above, carbocyclic aryl, heteroalicyclic or group;
and pharmaceutically acceptable salts thereof.

The invention also provides tetracycline-type compounds that are substituted by other than hydrogen at the 9-position. These compounds are generally referred to herein as 9-substituted tetracyclines, or simply 9-substituted compounds. Preferred 9-position

Thus, the 9-substituted tetracycline compounds of the invention have the following Formula II: 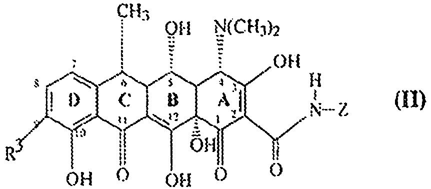
wherein R³ is alkyl preferably having 1 to about 20 carbon atoms, more preferably 1 to about 12 carbon atoms; alkenyl preferably having from 2 to about 20 carbon atoms, more preferably 2 to about 12 carbon atoms; alkynyl preferably having from 2 to about 20 carbon atoms, more preferably 2 to about 12 carbon atoms; alkoxy preferably having from 1 to about 20 carbon atoms, more preferably 1 to about 12 carbon atoms; alkylthio preferably having from 1 to about 20 carbon atoms, more preferably 1 to about 1 carbons atoms; alkylsulfinyl preferably having from 1 to about 20 carbon atoms, more preferably 1 to about 12 carbon atoms; alkylsulfonyl preferably having from 1 to about 20 carbon atoms, more preferably 1 to about 12 carbon atoms; or an alkyaryl such as benzyl;
Z is hydrogen, alkyl; alkenyl; alkynyl; alkoxy; alkylthio; alkylsulfinyl; alkylsulfonyl; arylalkyl; carbocyclic aryl; heteroalicyclic; an alkyl group having an amino group attached thereto; or heteroaromatic group;
and pharmaceutically acceptable salts thereof.

Compounds of the invention are active against susceptible microorganisms, including tetracycline-sensitive bacteria as well as tetracycline-resistant bacteria. Particularly preferred compounds of the invention exhibit 24-hour minimum inhibitory concentration (MIC) values of about 10 µg/ml or less, more preferably about 1 µg/ml or less, against tetracycline-resistant *E. coli. S. aureus* and *E. faecalis* strains such as *E. coli* D31m4(pHCMI), *S. aureus* RN4250 and *E. faecalis* pMV158. Preferred compounds of the invention also include those that exhibit such MIC values against tetracycline-sensitive *E. coli, S*. *aureus* and *E*. *faecalis* strains such as *E. coli* D31m4, *S. aureus* RN450 and *E_{.} faecalis* ATCC9790.

The invention thus enables methods of treatment against susceptible microorganisms such as bacteria, fungi, rickettsia, parasites and the like, and diseases associated with such microorganisms. These therapeutic methods in general comprise administration of a therapeutically effective amount of one or more compounds of the invention to a living subject that is suffering from or susceptible to infection by a susceptible microorganism such as bacteria, fungi, rickettsia and the like. Suitable subjects for treatment include animals, particularly a mammal such as human, or plants.

In an aspect, methods and compositions are provided for altering a cell from a tetracycline-resistant state to a tetracycline-sensitive state. In one preferred embodiment, these methods comprise the following steps: 1) administering to the cell a blocking agent that is a compound of invention and capable of interacting (e.g. binding) to a product of at least one tetracycline-resistance determinant capable of protecting ribosomes in the cell from tetracycline's inhibitory activity; and 2) concomitantly administering to the cell a pre-determined quantity of a tetracycline compound that is different than the blocking agent used in step 1. The cell then preferentially reacts with the blocking agent.

In another aspect, compounds of the invention are provided for use in the treament of infection by a susceptible microorganism such as bacteria, fungi, rickettsia and the like. In yet another aspect, compounds of the invention are provided in the manufacture of a medicament for the treament of infection by a susceptible microorganism such as bacteria, fungi, rickettsia and the like.

The invention further provides pharmaceutical compositions that comprise one or more compounds of the invention and a suitable carrier. Other aspects of the invention are disclosed *infra.*

### Detailed Description of the Invention

The present invention will be more fully illustrated by reference to the definitions set forth below.

"Tetracycline" is intended to include tetracycline and other tetracycline family members such as chlortetracycline, oxytetracycline, demeclocycline, methacycline, doxycycline, minocycline, etc. as well as other tetracycline compounds having the characteristic fused ring structure noted above in the Background Of The Invention.

The term "aliphatic group" is intended to include organic compounds characterized by straight or branched chains, typically having between 1 and 22 carbon atoms. Aliphatic groups include alkyl groups, alkenyl groups and alkynyl groups. In complex structures, the chains can be branched or cross-linked. Alkyl groups include saturated hydrocarbons having one or more carbon atoms, including straight-chain alkyl groups and branched-chain alkyl groups. Such hydrocarbon moieties may be substituted on one or more carbons with, for example, a halogen, a hydroxyl, a thiol, an amino, an alkoxy, an alkylcarboxy, an alkylthio, or a nitro group. Unless the number of carbons is otherwise specified, "lower aliphatic" as used herein means an aliphatic group, as defined above (e.g., lower alkyl, lower alkenyl, lower alkynyl), but having from one to six carbon atoms. Representative of such lower aliphatic groups, e.g., lower alkyl groups, are methyl, ethyl, n-propyl, isopropyl, 2-chloropropyl, n-butyl, sec-butyl, 2-aminobutyl, isobutyl, tert-butyl, 3-thiopentyl, and the like. As used herein, the term "amino" means -NH₂; the term "nitro" means -NO₂; the term "halogen" designates -F, - Cl, -Br or -I; the term "thiol" means SH; and the term "hydroxyl" means -OH. Thus, the term "alkylamino" as used herein means an alkyl group, as defined above, having an amino group, preferably 1 to about 3 or 4, attached thereto. Suitable alkylamino groups include groups having 1 to about 12 carbon atoms, preferably from 1 to about 6 carbon atoms. The term "alkylthio" refers to an alkyl group, as defined above, having a sulfhydryl group, preferably 1 to about 5 or 6, attached thereto. Suitable alkylthio groups include groups having 1 to about 12 carbon atoms, preferably from 1 to about 6 carbon atoms. The term "alkylcarboxyl" as used herein means an alkyl group, as defined above, having a carboxyl group attached thereto. The term "alkoxy" as used herein means an alkyl group, as defined above, having an oxygen atom, preferably 1 to 5, attached thereto. Representative alkoxy groups include groups having 1 to about 12 carbon atoms, preferably 1 to about 6 carbon atoms, e.g., methoxy, ethoxy, propoxy, tert-butoxy and the like. The terms "alkenyl" and "alkynyl" refer to unsaturated aliphatic groups analogous to alkyls, but which contain at least one double or triple bond respectively. Suitable alkenyl and alkynyl groups desirably have 1 to about 3 or 4 double or triple bonds and include groups having 2 to about 12 carbon atoms, preferably from 1 to about 6 carbon atoms.

The term "alicyclic group" is intended to include closed ring structures of three or more carbon atoms. Alicyclic groups include cycloparaffins or naphthenes which are saturated cyclic hydrocarbons, cycloolefins which are unsaturated with two or more double bonds, and cycloacetylenes which have a triple bond. They do not include aromatic groups. Examples of cycloparaffins include cyclopropane, cyclohexane, and cyclopentane. Examples of cycloolefins include cyclopentadiene and cyclooctatetraene. Alicyclic groups also include fused ring structures and substituted alicyclic groups such as alkyl substituted alicyclic groups. In the instance of the alicyclics such substituents can further comprise a lower alkyl, a lower alkenyl, a lower alkoxy, a lower alkylthio, a lower alkylamino, a lower alkylcarboxyl, a nitro, a hydroxyl, -CF₃, -CN, or the like.

The term "heterocyclic group" is intended to include closed ring structures in which one or more of the atoms in the ring is an element other than carbon, for example, nitrogen, sulfur, or oxygen. Heterocyclic groups can be saturated or unsaturated and heterocyclic groups such as pyrrole and furan can have aromatic character. They include fused ring structures such as quinoline and isoquinoline. Other examples of heterocyclic groups include pyridine and purine. Heterocyclic groups can also be substituted at one or more constituent atoms with, for example, a halogen, a lower alkyl, a lower alkenyl, a lower alkoxy, a lower alkylthio, a lower alkylamino, a lower alkylcarboxyl, a nitro, a hydroxyl, -CF₃, -CN, or the like. Suitable heteroaromatic and heteroalicyclic groups generally will have 1 to 3 separate or fused rings with 3 to about 8 members per ring and one or more N, O or S atoms, e.g. coumarinyl, quinolinyl, pyridyl, pyrazinyl, pyrimidyl, furyl, pyrrolyl, thienyl, thiazolyl, oxazolyl, imidazolyl, indolyl, benzofuranyl, benzothiazolyl, tetrahydrofuranyl, tetrahydropyranyl, piperidinyl, morpholino and pyrrolidinyl.

The term "aromatic group" is intended to include unsaturated cyclic hydrocarbons containing one or more rings. Aromatic groups include 5- and 6-membered single-ring groups which may include from zero to four heteroatoms, for example, benzene, pyrrole, furan, thiophene, imidazole, oxazole, thiazole, triazole, pyrazole, pyridine, pyrazine, pyridazine and pyrimidine, and the like. The aromatic ring may be substituted at one or more ring positions with, for example, a halogen, a lower alkyl, a lower alkenyl, a lower alkoxy, a lower alkylthio, a lower alkylamino, a lower alkylcarboxyl, a nitro, a hydroxyl, -CF₃, -CN, or the like.

The term "alkyl" refers to the saturated aliphatic groups, including straight-chain alkyl groups, branched-chain alkyl groups, cycloalkyl (alicyclic) groups, alkyl substituted cycloalkyl groups, and cycloalkyl substituted alkyl groups. In preferred embodiments, a straight chain or branched chain alkyl has 20 or fewer carbon atoms in its backbone (e.g., C₁-C₂₀ for straight chain, C₃-C₂₀ for branched chain), and more preferably 12 or fewer. Likewise, preferred cycloalkyls have from 4-10 carbon atoms in their ring structure, and more preferably have 4-7 carbon atoms in the ring structure. The term "lower alkyl" refers to alkyl groups having from 1 to 6 carbons in the chain, and to cycloalkyls having from 3 to 6 carbons in the ring structure.

Moreover, the term "alkyl" (including "lower alkyl") as used throughout the specification and claims is intended to include both "unsubstituted alkyls" and "substituted alkyls", the latter of which refers to alkyl moieties having substituents replacing a hydrogen on one or more carbons of the hydrocarbon backbone. Such substituents can include, for example, halogen, hydroxyl, alkylcarbonyloxy, arylcarbonyloxy, alkoxycarbonyloxy, aryloxycarbonyloxy, carboxylate, alkylcarbonyl, alkoxycarbonyl, aminocarbonyl, alkylthiocarbonyl, alkoxyl, phosphate, phosphonato, phosphinato, cyano, amino (including alkyl amino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfate, sulfonato, sulfamoyl, sulfonamido, nitro, trifluoromethyl, cyano, azido, heterocyclyl, aralkyl, or an aromatic or heteroaromatic moiety. It will be understood by those skilled in the art that the moieties substituted on the hydrocarbon chain can themselves be substituted, if appropriate. Cycloalkyls can be further substituted, e.g., with the substituents described above. An "aralkyl" moiety is an alkyl substituted with an aryl, e.g., having 1 to 3 separate or fused rings and from 6 to about 18 carbon ring atoms, (e.g., phenylmethyl (benzyl)).

The term "alkoxy", as used herein, refers to a moiety having the structure -O-alkyl, in which the alkyl moiety is described above.

The term "aralkoxy", as used herein, refers to a moiety having the structure -O-aralkyl, in which the aralkyl moiety is described above. Suitable aralkoxy groups have 1 to 3 separate or fused rings and from 6 to about 18 carbon ring atoms, with O-benzyl being a preferred group.

The term "aryl" as used herein includes 5- and 6-membered single-ring aromatic groups that may include from zero to four heteroatoms, for example, unsubstituted or substituted benzene, pyrrole, furan, thiophene, imidazole, oxazole, thiazole, triazole, pyrazole, pyridine, pyrazine, pyridazine and pyrimidine, and the like. Aryl groups also include polycyclic fused aromatic groups such as naphthyl, quinolyl, indolyl, and the like. The aromatic ring can be substituted at one or more ring positions with such substituents, e.g., as described above for alkyl groups. Preferred aryl groups include unsubstituted and substituted phenyl groups.

The term "aryloxy", as used herein, refers to a group having the structure -O-aryl, in which the aryl moiety is as defined above.

The term "amino," as used herein, refers to an unsubstituted or substituted moiety of the formula -NRₐR_{b}, in which Rₐ and R_{b} are each independently hydrogen, alkyl, aryl, or heterocyclyl, or Rₐ and R_{b}, taken together with the nitrogen atom to which they are attached, form a cyclic moiety having from 3 to 8 atoms in the ring. Thus, the term "amino" is intended to include cyclic amino moieties such as piperidinyl or pyrrolidinyl groups, unless otherwise stated. An "amino-substituted amino group" refers to an amino group in which at least one of Rₐ and R_{b}, is further substituted with an amino group.

Alkylsulfinyl groups have one or more sulfonyl (SO) linkages, typically 1 to about 5 or 6 sulfinyl linkages. Suitable alkylsulfinyl groups include groups having I to about 12 carbon atoms, preferably from 1 to about 6 carbon atoms.

Alkylsulfonyl groups have one or more sulfonyl (SO₂) linkages, typically 1 to about 5 or 6 sulfonyl linkages. Suitable alkylsulfonyl groups include groups having 1 to about 12 carbon atoms, preferably from 1 to about 6 carbon atoms.

Suitable alkanoyl groups include groups having 1 to about 4 or 5 carbonyl groups. Suitable aroyl groups include groups having one or more carbonyl groups as a substituent to an aryl group such as phenyl or other carbocyclic aryl. Suitable alkaroyl groups have one or more alkylcarbonyl groups as a substituent to an aryl group such as phenylacetyl and the like. Suitable carbocyclic aryl groups have 6 or more carbons such as phenyl, naphthyl and the like. Suitable aryloyl groups are carbocyclic aryl groups that are substituted with one or more carbonyl groups, typically 1 or 2 carbonyl groups.

Compounds of the invention can be used to treat against microorganisms, particularly gram-positive as well as some gram-negative bacteria. Preferred compounds include those of Formula I and II: 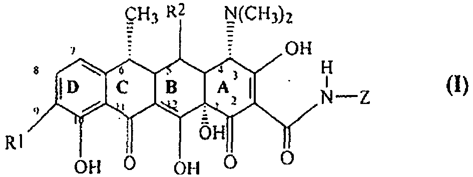 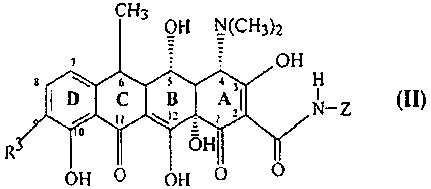 wherein R¹, R², R³ and Z are as defined above; and pharmaceutically acceptable salts of those compounds.

Compounds of the invention include 5-propionate-9-t-butyl doxycycline; 9-chloro-t-butyl-5-propionate doxycycline; 9-t-butyl-6-alpha-deoxy-5-formyloxy-tetracycline; 9-t-butyl-6-alpha-deoxy-5-acetoxy-tetracycline; 9-t-butyl-6-alpha-deoxy-5-propionyloxy-tetracycline; 9-t-butyl-6-alpha-deoxy-5-phenylcarbonyloxy-tetracycline; 9-t-butyl-6-alpha-deoxy-5-benzylcarbonyloxy-tetracycline; 9-t-butyl-6-alpha-deoxy-5-dimethylaminocarbonyloxy-tetracycline; 9-t-butyl-6-alpha-deoxy-5-cyclopentylcarbonyloxy-tetracycline; 9-t-butyl-6-alpha-deoxy-5-cyclobutylcarbonyloxy-tetracycline; 9-t-butyl-6-alpha-deoxy-5-cyclohexylcarbonyloxy-tetracycline; 9-t-butyl-6-alpha-deoxy-5-cycloheptylcarbonyloxy-tetracycline; 9-(chloro-t-butyl)-6-deoxy-oxytetracycline, 9-[(dimethylamino)-t-butyl]-6-deoxy-oxytetracycline, 9-(amino)-t-butyl-6-deoxy-oxytetracycline, 9-[(piperidino)-t-butyl]-6-deoxy-oxytetracycline, 9-[(diethylamino)-t-butyl]-6-deoxy-oxytetracycline, 9-[(dipropylamino)-t-butyl]-6-deoxy-oxytetracycline, 9-[(dimethylamino)-t-butyl]-6-alpha-deoxy-5-formyloxy-tetracycline; 9-[(dimethylamino)-t-butyl]-6-alpha-deoxy-5-acetoxy-tetracycline; 9-[(dimethylamino)-t-butyl]-6-alpha-deoxy-5-propionylcarbonyloxy-tetracycline; 9-[(dimethylamino)-t-butyl]-6-alpha-deoxy-5-phenylcarbonyloxy-tetracycline; 9-[(dimethylamino)-t-butyl]-6-alpha-deoxy-5-benzylcarbonyloxy-tetracycline; 9-[(dimethylamino)-t-butyl]-6-alpha-deoxy-5-dimethylaminocarbonyloxy-tetracycline; 9-[(dimethylamino)-t-butyl]-6-alpha-deoxy-5-cyclopentylcarbonyloxy-tetracycline; 9-[(dimethylamino)-t-butyl]-6-alpha-deoxy-5-cyclobutylcarbonyloxy-tetracycline; 9-[(dimethylamino)-t-butyl]-6-alpha-deoxy-5-cyclohexylcarbonyloxy-tetracycline; 9-[(dimethylamino)-t-butyl]-6-alpha-deoxy-5-cycloheptylcarbonyloxy-tetracycline; and pharmaceutically, acceptable salts thereof.

Particularly preferred compounds of the invention include 5-propionate-9-t-butyl doxycycline; 9-t-butyl-6-deoxy-5-hydroxytetracycline, 9-t-buryl-6-deoxy-5-ropionylcarbonyloxytetracycline, 9-t-butyl-6-deoxy-5-acetylcarbonyloxytetracycline, 9-t-butyl-6-deoxy-5-cyclobutylcarbonyloxytetracycline, 9-[1'-(1'-methyl)cyclohexyl]-6-deoxy-5-hydroxytetracycline, 9-[1'-(1'-methyl)cyclopentyl]-6-deoxy-5-hydroxytetracycline, 9-[1'-(1'-methyl)cyclobutyl]-6-deoxy-5-hydroxytetracycline, 9-[2'-(2'-methyl)pentyl]-6-deoxy-5-hydroxytetracycline, 9-[4'-(1'-bromo-4'-methyl)pentyl]-6-deoxy-5-hydroxytetracycline, 9-[4'-(1'-dimethylamino-4'-methyl)pentyl]-6-deoxy-5-hydroxytetracycline, 9-[4'-(1'-pyrrolidinyl-4'-methyl)pentyl]-6-deoxy-5-hydroxytetracycline, 9-[4'-(1'-cyano-4'-methyl)pentyl]-6-deoxy-5-hydroxytetracycline, 9-[4'-(1'-nitro-4'-methyl)pentyl]-6-deoxy-5-hydroxytetracycline, 9-[4'-(1'-acetoxy-4'-methyl)pentyl]-6-deoxy-5-hydroxytetracycline); and pharmaceutically acceptable salts thereof.

Compounds of the invention can be prepared as generally depicted in the following Schemes I and II. In the discussions of the Schemes, the various substituent groups are the same as defined above for Formulas I and II. Also, for purposes of exemplification only, doxycycline is depicted as the "base" tetracycline compound, although it will be understood that a wide variety of tetracycline compounds can be employed in the same manner. For example, the base tetracycline compound substituted at the 5- and/or 9-positions suitably may be oxytetracycline; chlortetracycline; demeclocycline; doxycycline; chelocardin; minocycline; roliteteracycline; lymecycline; sancycline; methacycline; apicycline; clomocycline; guamecycline; meglucycline; mepylcycline; penimepicycline; pipacycline; etamocycline; penimocycline and the like.

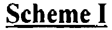

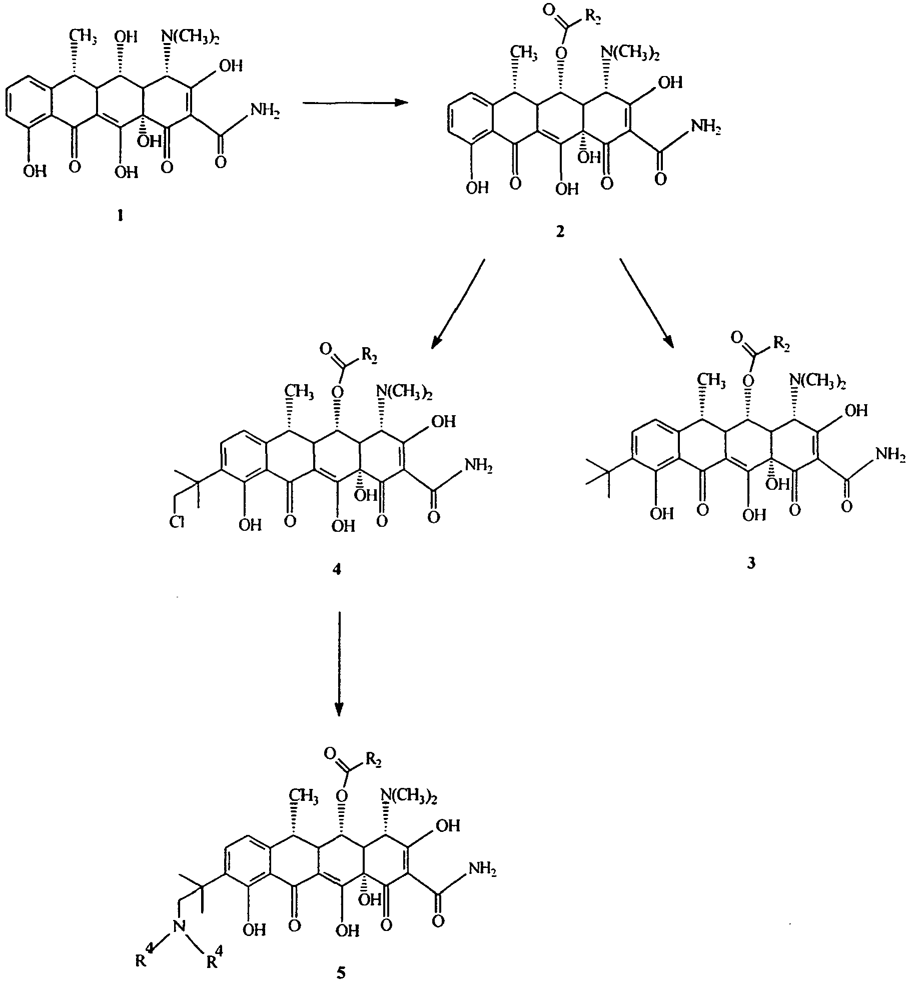

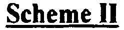

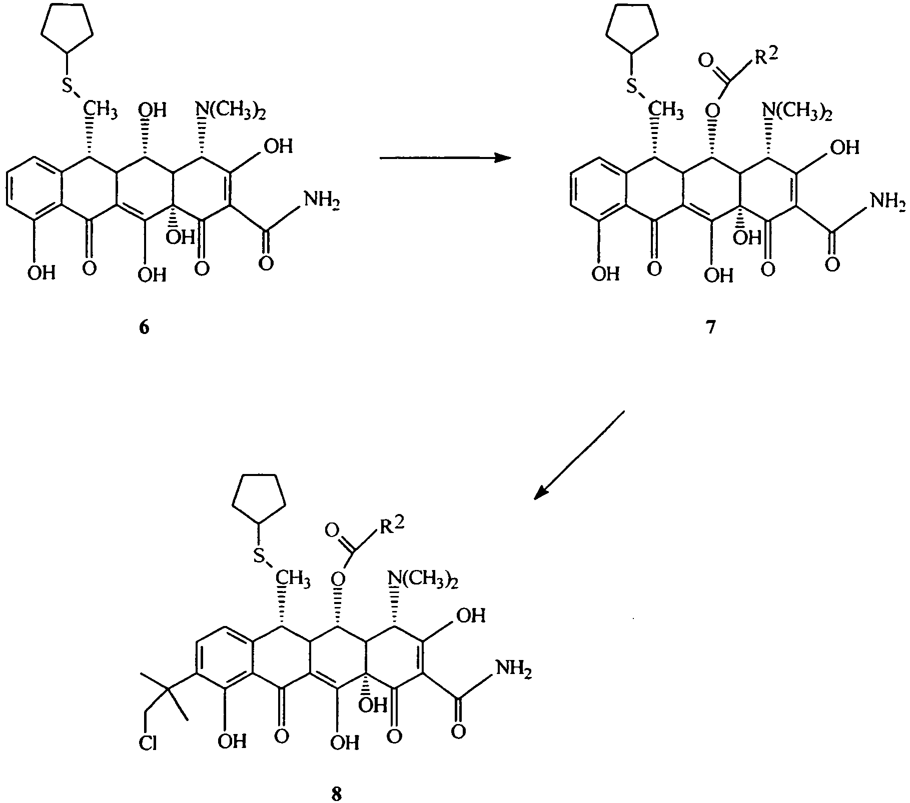

As shown in Scheme I above, the tetracycline base compound 1 (i.e. depicted as doxycycline or alpha-6-deoxy-5-oxytetracycline) is suitably first substituted at the 5-position such as by functionalization of the depicted 5-hydroxy group to form a 5-position ester by reacting compound 1 with a compound R²CO₂H in the presence of acid, such as anhydrous HF, trifluoromethanesulfonic acid and methanesulfonic acid at temperatures suitably ranging between 20° and 100°C. See the examples which follow for exemplary reactions. See also U.S. Patent 5,589,470 for a discussion of preparation of C₅ esters. 5-position ethers can be suitably formed by reaction of compound 1 with an alkylating agent such as an alkyl halide, or other reactive agent.

The 5-substituted tetracycline compound 2 then can be reacted with a cation-forming species such as t-butanol or 1-chloro-2-methyl propene in a strong acid such as methanesulfonic acid suitably at temperatures of 20° to 100°C. in a Friedel-Crafts-type reaction to provide 5,9-substituted compounds of the invention such as compounds 3 and 4 depicted in Scheme I and compound 8 in Scheme II. Compounds 4 and 8 can be further functionalized at the 9-position by reaction with appropriate nucleophilic reagent such as compounds of the formula X-(R⁴)_{1 or 2} where X is heteroatom such as N, O or S and each R⁴ is independently e.g. C₁₋₁₂alkyl, aryl particularly carbocyclic aryl such as phenyl, etc.

As discussed above, the invention provides methods of treatment against microorganism infections and associated diseases, which methods in general will comprise administration of a therapeutically effective amount of one or more compounds of the invention to a subject, which may be an animal or plant, and typically is a mammal, preferably a primate such as a human.

As further discussed above, the invention also provides methodology to overcome resistance of the ever-increasing varieties of cells and microorganisms to known tetracyclines. This methodology in general comprises the steps of 1) administering to the cell a blocking agent that is a compound of invention and capable of interacting (e.g., binding) to a product of at least one tetracycline resistance determinant capable of protecting ribosomes in the cell from tetracycline's inhibitory activity; and 2) concomitantly (i.e. simultaneously or sequentially) administering to the cell a pre-determined quantity of a tetracycline compound that is different than the blocking agent used in step 1. The resistance mechanism of the cell is allowed to preferentially react with the blocking agent (i.e. the compound of the invention) to avoid preferential reaction with the second administered composition (i.e. the tetracycline compound that is different than the blocking agent).

This methodology takes into account and acts upon the existence of specific DNA sequences, which are typically found on plasmids and transposons, and which specify proteins for tetracycline-resistance determinants. Some of these determinants act via an active efflux system which maintains an intracellular tetracycline concentration below those levels able to inhibit protein within the microorganism such as described in U.S. Patents 4,806,529 and 5,589,470. Other determinants act by protecting the ribosome from tetracycline's inhibitory activity, e.g. by binding with tetracycline. The methodology utilizes a compound of the invention as a blocking agent to interact with a product of at least one tetracycline resistance determinant which acts by protecting the cell from tetracycline's inhibitory activity. The determinant is capable of making a product, such as a cytoplasmic protein, which interacts with the ribosomes to make them tetracycline resistant or a membrane protein which keeps tetracycline out of a cell.

This methodology is particularly suitable for use with tetracycline-resistant cells or organisms which contain or carry a product of the genetic determinants responsible for tetracycline resistance, and in particular, those which are due to protection of the ribosome from the inhibitory activity of tetracycline. As disclosed in Levy. S.B., Journal of Antimicrobial Chemotherapy, 24:1-3 (1989), more than a dozen different distinguishable tetracycline resistance determinants have been uncovered. See also Levy, S.B., "Resistance to the Tetracyclines," in Antimicrobial Drug Resistance, (Bryan, L.E., editor), Academic Press, Orlando, Florida, 1984, pages 191-204; and Levy, S.B., ASM News, 54:418-421 (1988). As these genetic determinants of these tetracycline-resistant cells have been elucidated, it has become generally accepted that the same or very similar genes are responsible for resistance in a large number of different aerobic and anaerobic microorganisms.

This methodology of invention is therefore believed suitable for use with at least, but not exclusively, the following genera: Gram-negative genera, in particular *Enterobacteriaceae,* which harbor Class A-E tetracycline resistance determinants; Gram-positive genera including streptococci, Staphylococci, and bacillus species which bear the Class K and L tetracycline resistance determinants; aerobic and anaerobic microorganisms bearing the Class M, O or Q determinants represented by *Streptococcus agalactiae, Bacteroides, Enterococcus, Gardnerella* and *Neisseria* species, Mycoplasma and Ureaplasma, and *Clostridium; Clostridiumperfringens* bearing the Class P tetracycline-resistant determinant.

Examples of products of a tetracycline resistance determinant are Tet M, Tet 0 and Tet Q proteins for cytoplasmic protein products and Tet A, Tet B, Tet K and Tet L for membrane products.

It will be recognized and appreciated that the above listed organisms are themselves only representative and illustrative of the range, variety, and diversity of cell types, bacterial species, fungi, parasites, and rickettsial disease agents which may be therapeutically treated using the methods of the invention. No specific class, genus, species, or family of cell, microorganism, or parasite is excluded from treatment by the methods of the invention. Indeed, it is expected that with future investigations into the determinants responsible for tetracycline resistance, ever greater numbers of different cells will be recognized as suitable for efficacious treatment using the present invention. In addition, in view of the recent use of tetracyclines for treatment of neoplasms, it is deemed that the present methodology would be useful in such therapies (van der Bozert et al., Cancer Res., 48:6686-6690 (1988)).

As discussed above, in this methodology, two different compositions are administered concurrently, sequentially or simultaneously to a tetracycline-resistant cell. Moreover, this methodology requires and relies upon a preferential binding and reaction with the administered blocking agent *in situ;* and consequently demonstrates a substantial lack of attraction or preference for the other administered tetracycline compound. The operation, utility, and efficacy of the present methodology is thus based upon an empirically demonstrable preference of the tetracycline-resistant cell for one class of composition over another when both classes of composition are introduced concomitantly, i.e. concurrently, sequentially or simultaneously to the resistant cell.

The second tetracycline compound that is administered with the blocking agent may be any "tetracycline-type" compound currently known which includes tetracycline itself; or any member of the tetracycline family that is distinct from the administered blocking agent. Suitable compounds are disclosed e.g. in U.S. 5,589,470 to Levy, including compounds of Formula III as set forth at columns 8-9 of that patent, and more specifically suitable compounds include tetracycline, oxytetracycline; chlortetracycline; demeclocycline; doxycycline; chelocardin; minocycline; rolitetracycline; lymecycline; sancycline, methacycline; apicycline; chlomocycline; guamecycline; meglucycline; mepycycline; penimepicycline; pipacycline; etamocycline; and penimocycline. Other suitable agents are described within Essentials of Medicinal Chemist!)', John Wiley and Sons, Inc., 1976, pages 512-517.

One or more compounds of the invention may be administered alone to a subject, or more typically a compound of the invention will be administered as part of a pharmaceutical composition in mixture with conventional excipient, i.e., pharmaceutically acceptable organic or inorganic carrier substances suitable for parenteral, oral or other desired administration and which do not deleteriously react with the active compounds and are not deleterious to the recipient thereof. Suitable pharmaceutically acceptable carriers include but are not limited to water, salt solutions, alcohol, vegetable oils, polyethylene glycols, gelatin, lactose, amylose, magnesium stearate, talc, silicic acid, viscous paraffin, perfume oil, fatty acid monoglycerides and diglycerides, petroethral fatty acid esters, hydroxymethylcellulose, polyvinylpyrrolidone, etc. The pharmaceutical preparations can be sterilized and if desired mixed with auxiliary agents, e.g., lubricants, preservatives, stabilizers, wetting agents, emulsifiers, salts for influencing osmotic pressure, buffers, colorings, flavorings and/or aromatic substances and the like which do not deleteriously react with the active compounds.

At least many of the compounds of the invention suitably may be administered to a subject in a protonated and water-soluble form, e.g., as a pharmaceutically acceptable salt of an organic or inorganic acid, e.g., hydrochloride, sulfate, hemi-sulfate, phosphate, nitrate, acetate, oxalate, citrate, maleate, mesylate, etc. Also, where an appropriate acidic group is present on a compound of the invention, a pharmaceutically acceptable salt of an organic or inorganic base can be employed such as an ammonium salt, or salt of an organic amine, or a salt of an alkali metal or alkaline earth metal such as a potassium, calcium or sodium salt.

Therapeutic compounds can be administered to a subject in accordance with the invention by any of a variety of routes. Topical (including transdermal, buccal or sublingual), and parenteral (including intraperitoneal, subcutaneous, intravenous, intradermal or intramuscular injection) are generally preferred.

For parenteral application, particularly suitable are solutions, preferably oily or aqueous solutions as well as suspensions, emulsions, or implants, including suppositories. Therapeutic compounds will be formulated in sterile form in multiple or single dose formats such as being dispersed in a fluid carrier such as sterile physiological saline or 5% saline dextrose solutions commonly used with injectables.

For enteral application, particularly suitable are tablets, dragees or capsules having talc and/or carbohydrate carrier binder or the like, the carrier preferably being lactose and/or corn starch and/or potato starch. A syrup, elixir or the like can be used wherein a sweetened vehicle is employed. Sustained release compositions can be formulated including those wherein the active component is protected with differentially degradable coatings, e.g., by microencapsulation, multiple coatings, etc.

For topical applications, the compound(s) can be suitably admixed in a pharmacologically inert topical carrier such as a gel, an ointment, a lotion or a cream. Such topical carriers include water, glycerol, alcohol, propylene glycol, fatty alcohols, triglycerides, fatty acid esters, or mineral oils. Other possible topical carriers are liquid petrolatum, isopropylpalmitate, polyethylene glycol, ethanol 95%, polyoxyethylene monolauriate 5% in water, sodium lauryl sulfate 5% in water, and the like. In addition, materials such as anti-oxidants, humectants, viscosity stabilizers and the like also may be added if desired.

In addition to treatment of humans, the methods of the invention also will have significant veterinary applications. e.g. for treatment of livestock such as cattle, sheep, goats, cows, swine and the like; poultry such as chickens, ducks, geese, turkeys and the like; horses; and pets such as dogs and cats.

It will be appreciated that the actual preferred amounts of active compounds used in a given therapy will vary according to the specific compound being utilized, the particular compositions formulated, the mode of application, the particular site of administration, etc. Optimal administration rates for a given protocol of administration can be readily ascertained by those skilled in the art using conventional dosage determination tests conducted with regard to the foregoing guidelines.

In general, compounds of the invention for treatment can be administered to a subject in dosages used in prior tetracycline therapies. See, for example, the *Physicians' Desk Reference.* For example, a suitable effective dose of one or more compounds of the invention will be in the range of from 0.01 to 100 milligrams per kilogram of body weight of recipient per day, preferably in the range of from 0.1 to 50 milligrams per kilogram body weight of recipient per day, more preferably in the range of 1 to 20 milligrams per kilogram body weight of recipient per day. The desired dose is suitably administered once daily, or several sub-doses, e.g. 2 to 5 sub-doses, are administered at appropriate intervals through the day, or other appropriate schedule.

With respect to the particular methods of the invention where a compound of the invention is used as a blocking agent and administered in conjunction with a distinct tetracycline compound, the general molar ratio of the blocking agent (i.e. compound of the invention) to the other tetracycline compound suitably will be from about 0.01:100, and preferably from 0.05:2.0. It is preferred that the blocking agent is administered in concentrations that that are in excess of MIC levels of about 1,000 µg/ml, and that the other or second tetracycline compound is administered in accordance with conventional practice for efficacious therapeutic treatment of infection or disease in humans or other animals. See, e.g., the *Physicians' Desk Reference.*

It will also be understood that normal, conventionally known precautions will be taken regarding the administration of tetracyclines generally to ensure their efficacy under normal use circumstances. Especially when employed for therapeutic treatment of humans and animals *in vivo,* the practitioner should take all sensible precautions to avoid conventionally known contradictions and toxic effects. Thus, the conventionally recognized adverse reactions of gastrointestinal distress and inflammations, the renal toxicity, hypersensitivity reactions, changes in blood, and impairment of absorption through aluminum, calcium, and magnesium ions should be duly considered in the conventional manner.

The present invention is further illustrated by the following examples. These examples are provided to aid in the understanding of the invention and are not to be construed as limitations thereof. Example 1 does not form part of the invention.

### Example 1

### 6-alpha-deoxy-5-propionylcarbonyloxy-tetracycline

5-esters of doxycycline were prepared according to the known procedure. Thus doxycycline, (500 mg, 1mmol) was dissolved in cold anhydrous HF (25 mL) at 0°C. To it was added propionic acid (4 mL, 3.97 g. 54 mmol). The resulting mixture was left overnight at room temperature. The HF was slowly evaporated under a steady nitrogen stream, and the residue dissolved in methanol. The solution was dried *in vacuo* to produce a crude yellow solid. The solid was treated with activated charcoal (2g), filtered, and subjected to C18 reverse-phase column chromatography. Isolation of fractions containing the compound were pooled, the butanol layer was washed once with brine and twice with distilled water to afford the compound as a yellow product. MS(FAB: m/z 501).

### Example 2

### 9-t-butyl-6-alpha-deoxy-5-tetracycline

Doxycycline was dissolved in 1 mL of t-butanol and 2 mL methanesulfonic acid. The solution was stirred overnight at room temperature. The reaction mixture was poured into ice water (50mL), the pH brought to 5.5 with dilute NaOH, and the precipitate collected by filtration. The crude solid was extracted into CHCl₃, treated with Na₂SO₄, filtered, and the solvent removed *in vacuo.* A yellow solid was obtained by C₁₈ reverse-phase preparative column chromatography that was followed by extraction into butanol. The butanol layer was washed once with brine and twice with distilled water. Concentration *in vacuo* gave 9-t-butyl doxycycline as a yellow solid. The HCl salt was produced by dissolving the compound in methanol and bubbling gaseous HCl until saturated. Removal of the solvent led to production of yellow crystals. MS(FAB:m/z 501).

### Example 3

### 9-t-butyl-6-apha-deoxy-5-propionylcarbonyloxy-tetracycline

The product of Example 1 above (100 mg. 2 mmol) was dissolved in 1 ml of t-butanol and 2 mL methanesulfonic acid. The solution was stirred overnight at room temperature. The reaction mixture was poured into ice water (50 mL), the pH brought to 5.5 with dilute NaOH, and the precipitate collected by filtration. The solid was purified as in Example 2 above and the HCl salt produced a yellow solid. MS(FAB:m/z 557).

### Example 4

### 9-t-butyl-6-alpha-deoxy-5-cyclobutanylcarbonyloxy-tetracycline

A product produced similar to Example 1 (using cyclobutanoic acid as the carboxylic acid) (100 mg, 2 mmol) was dissolved in 1 ml of t-butanol and 2 mL methanesulfonic acid. The solution was stirred overnight at room temperature. The reaction mixture was poured into ice water (50mL), the pH brought to 5.5 with dilute NaOH, and the precipitate collected by filtration. The solid was purified as in Example 2 above and the HCl salt produced a yellow solid. MS(FAB:m/z 582).

### Example 5

### 9-(chloro-t-butyl)-6-alpha-deoxy-5-oxy-tetracycline

Doxycycline (400mg, .9mmol) was dissolved in 3mL of 1-chloro-2-methyl-2-propanol or 2-methyl-1-chloropropene and 4 mL methanesulfonic acid. The solution was heated to 45°C under a nitrogen atmosphere for 30 hrs. The reaction mixture was poured into ice water (50 mL), the pH brought to 5.5 with dilute NaOH, and the precipitate collected by filtration. The crude solid was extracted into CHCl₃, treated with Na₂SO₄, filtered, and the solvent removed *in vacuo.* A yellow solid was obtained by C₁₈ reverse-phase preparative column chromatography that was followed by extraction into butanol. The butanol layer was washed once with brine and twice with distilled water. Concentration *in vacuo* gave 9-(chloro-t-butyl) doxycycline as a yellow solid. MS(FAB:m/z M+H 535, M+2, 537).

### Example 6

### 9-[(piperidino)t-butyl]-6-alpha-deoxy-5-oxy-tetracycline

Product of Example 5 above (100 mg, 0.2 mmol) was dissolved in 1 -methyl-2-pyrrolidinone and 2 equivalents of piperidine (14.8 ul), were stirred under nitrogen for 30 minutes. The solvent was removed *in vacuo* and the residue dissolved in methanol. The residue was precipitated with diethyl ether, filtered, and the solid collected. The compound was obtained as a yellow glass by C₁₈ reverse-phase column chromatography. MS(FAB:m/z M+H 584).

### Example 7

### 9-[(dimethylamine)t-butyl]-6-alpha-deoxy-5-oxy-tetracycline

Product of Example 5 above 100mg. 0.2 mmol) was dissolved in 1-methyl-2-pyrrolidinone and 2 equivalents of dimethylamine (14.8 ul), were stirred under nitrogen for 30 minutes. The solvent was removed *in vacuo* and the residue dissolved in methanol. The residue was precipitated with diethyl ether, filtered, and the solid collected. The compound was obtained as a yellow glass by C₁₈ reverse-phase column chromatography. MS(FAB:m/z M+H 544).

### Example 8

### 9-(chloro)t-butyl-6-alpha-deoxy-5-propionylcarbonyloxy-tetracycline

Product of Example 1 above (100 mg, 0.2 mmol) was dissolved in 2 mL of 2-methyl-1-chloropropene and 2 mL methanesulfonic acid. The solution was heated to 45°C under a nitrogen atmosphere for 10 hrs. The reaction mixture was poured into ice water (10mL), the pH brought to 5.5 with dilute NaOH, and the precipitate collected by filtration. The crude solid was extracted into CHCl₃, treated with Na₂SO₄, filtered, and the solvent removed *in vacuo.* A yellow solid was obtained by C₁₈ reverse-phase preparative column chromatography that was followed by extraction into butanol. The butanol layer was washed once with brine and twice with distilled water. Concentration *in vacuo* gave 9-(chloro)t-butyl-6-alpha-deoxy-5-propionylcarbonyloxy-tetracycline as a yellow solid.

### Example 9

### 9-(piperidino)t-butyl-6-alpha-deoxy-5-propionylcarborzyloxy-tetracycline

Product of Example 7 above (100 mg, 0.2 mmol) was dissolved in 1-methyl-2-pyrrolidinone and 2 equivalents of piperidine (14.8 uµl), were stirred under nitrogen for 30 minutes. The solvent was removed *in vacuo* and the residue dissolved in methanol. The residue was precipitated with diethyl ether, filtered, and the solid collected. The compound was obtained as a yellow glass by C₁₈ reverse-phase column chromatography.

### Example 10

The inhibitory effects of compounds of the invention were examined relative to doxycycline using tetracycline-sensitive and tetracycline-resistant strains of *E. coli, S. aureus,* and *E. faecalis* as specified in the Tables below. The general protocol for performing these experiments was as follows: cultures were grown up fresh in L broth in the morning from an overnight culture. After 4-6 hours of growth, each bacterial culture was diluted to an A₅₃₀ of 0.2-0.5 depending on the strain (*E. coli,* 0.5; *S. aureus, 0.4; E. faecalis,* 0.2). Individual tubes, containing 1 ml of L broth and different concentrations of the tested compound, were inoculated with the different bacterial cultures and then incubated at 37°C. After 17-18 hours of incubation, the concentration of the tested compound at which no observed cloudiness was seen was called the minimal inhibitory concentration (MIC). The results obtained are set forth in Tables 2 and 3 below, with the MIC values expressed in µg/ml and set forth in columns beneath the tested compound.

**Table 2**

| Comparison of Minimum Inhibitory Concentrations for Doxycycline and 5-propionyl-9-t-butyl Doxycycline Against Tetracycline Resistant and Sensitive Bacteria | | | |
|---|---|---|---|
| | 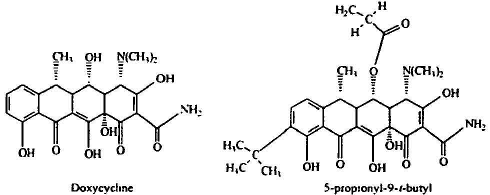 | | |

| Tetracycline Resistant Bacteria | | | |
|---|---|---|---|
| Strain | | | |
| E. coli | D31m4(pHCM1)* | (B) 12.5 | 1.56 |
| S. aureus | RN4250 | (K) 12.5 | 0.19 |
| S. aureus | 12715 | (K) 25 | 0.39 |
| E. faecalis | pMV158 | (L) 12.5 | ≤0.09 |
| E. faecalis | pAM211 | (L) 12.5 | 0.19 |

| Tetracycline Sensitive Bacteria | | | |
|---|---|---|---|
| Strain | | | |
| E. coli | D31m4* | <0.78 | 3.12 |
| S. aureus | RN450 | <0.78 | 0.19 |
| E. faecalis | ATCC9790 | <0.78 | 0.19 |

| | | | |
|---|---|---|---|
| * lipopolysaccharide-deficient mutant | | | |

**Table 3**

| Comparison of Minimum Inhibitory Concentrations for Doxycycline and 5-proplonyl-9-t-butyl Doxycycline Against Tetracycline Resistant and Sensitive Bacteria | | | |
|---|---|---|---|
| 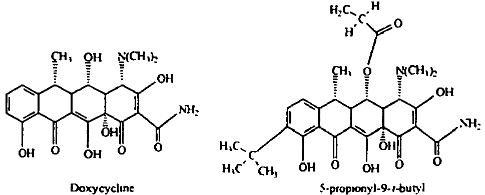 | | | |

| Doxycycline | | | |
|---|---|---|---|
| Tetracycline Resistant Bacteria | | | |
| MRSA | 1 | 6.25 | 1.56 (synergy) |
| MRSA | 3 | 6.25 | 0.78 |
| VRE | 11 | 12.5 | 0.78 (synergy) |
| VRE | 15 | 12.5 | 0.78 |
| | | | 0.78 |
| | | | |

| Tetracycline Sensitive Bacteria | | | |
|---|---|---|---|
| MRSA | 1 | 6.25 | 1.56 (synergy) |
| VRE | 17 | ≤1.56 | 0.78 |
| VRE | 19 | ≤1.56 | 0.39 |

| | | | |
|---|---|---|---|
| MRSA=methicillin resistant S. aureus, VRE=vancomycin resistant Enterococcus | | | |

Further compounds in accordance with the invention were made and evaluated as follows.

### Example 11

### [4S-(4alpha, 12aalpha)]-9-(tert-butyl)-4-(dimethylamino)-1,4,4a,5a,6,11,12a-octahydro-3.5,10,12,12a-pentahydroxy-6-methyl-1.11-dioxo-2-naphthacenecarboxamide

### (9-t-butyl-6-deoxy-5-hydroxytetracycline)

To a room temperature solution of 0.100 g of doxycycline hydrochloride in 2 ml of methanesulfonic acid is added I ml of tert butyl alcohol. The reaction is stirred for 18 hours under ambient atmosphere. The mixture is then poured into 40 ml of ice water and the resulting solid is extracted with chloroform and dried to afford 80 mg of the desired product as a yellow glass.

MS(FAB): m/z 501 (M+H).

¹H NMR (CD₃OD): δ 7.50(d,1H,J=8.07Hz,H-8); 6.86(d1H,J=8.07Hz,H-7); 4.44(bs,1H,H-4); 3.62(dd,1H, J=11.42; 8.35 Hz,H-5); 2.95(bs,6H, NMe₂); 2.81 (d,1H,J=11.45Hz, H-4a); 2.71 (dq, 1H, J=12.41; 6.5 Hz, H-6); 2.53 (dd,1H, J=12.23;8.20 Hz, H-5a); 1.51(d, 3H, J=6.78 Hz, CH₃); 1.41(bs, 9H, CMe₃).

### Example 12

### [4S-(4alpha,12aalpha)]-9-(tert-buty/)-4-(dimethylamino)-5-propionyl-1,4,4a,5,5a,6,11,12a-octahydro-3,10,12,12a-tetrahydroxy-6-methyl-1,11-dioxo-2-naphthacenecarboxamide (9-t-butyl-6-deoxy-5-propionylcarbonyloxytetracycline)

To a polypropylene tube containing 0.1 g of doxycycline is added 1 ml of propionic acid in excess. The solution is stirred and cooled in dry ice for 10 minutes followed by careful addition of 2 ml of anhydrous hydrofluoric acid. After 90 minutes, the acid is evaporated off to give the ester as a yellow glass. The ester is used without further purification to prepare the title compound according to the procedure in Example 11. Thus, 0.1 g of ester is dissolved in 2 ml of methanesulfonic acid and 1 ml of tert butyl alcohol is added. The reaction is stirred at room temperature and under ambient atmosphere for 18 hours, then poured over ice and extracted with chloroform. The extract is dried to afford the desired product as a yellow glass.

MS(FAB): m/z 557 (M+H).
¹H NMR (CD₃0D): d 7.54(d,1H,J=8.08Hz,H-8); 6.88(d,1H,J=8.08HzH-7); 5.16(did, J=10.44;7.94 Hz, H-5); 4.44(bs,1H,H-4); 3.74(d, 1H, J=2.07 Hz, H-4); 3.04(bs,6H, NMe₂); 2.90(dd,1H,J=7.94;2.07 Hz, H-4a); 2.72(dq, 1H, J=12.31; 6.56 Hz, H-6); 2.61(dd,1H, J= 12.31; 10.44 Hz, H-5a); 2.54(q, 2H. J=7.48 Hz, CH₂-C); 1.44(bs, 9H. CMe₃); 1.29(d, 3H, J=6.56 Hz, CH₃); 1.20(t, 3H, J=7.48 Hz, C-CH₃).

### Example 13

### [4S-(4alpha,12aalpha)]-9-(tert-butyl)-4-(dimethylamino)-5-acetylcarbonyloxy-1,4,4a,5a,6,11,12tahydro-3,10,12,12a-tetrahydroxy-6-methyl-1.11-dioxo-2-naphthacenecarboxamide (9-t-butyl-6-deoxy-5-aceylcarbonyloxytetracycline)

To a polypropylene tube containing 0.2 g of doxycycline is added 2 ml of glacial acetic acid in excess. The solution is stirred and cooled in dry ice for 10 hours followed by careful addition of 5 ml of anhydrous hydrofluoric acid. After 24 minutes, the acid is evaporated off under a slow, steady stream if nitrogen to give the 5-ester as a yellow glass. The ester is used without further purification to prepare the title compound according to the procedure in Example 11. Thus, 0.1 g of ester is dissolved in 2 ml of methanesulfonic acid and 1 ml of tert butyl alcohol is added. The reaction is stirred at room temperature and under ambient atmosphere for 18 hours, then poured over ice and extracted with chloroform. The extract is dried and the residue subjected to preparative HPLC to afford the desired product as a yellow glass.

MS(FAB): m/z 543 (M+H).
¹H NMR (CD₃OD): d 7.55(d,1H,J=8.08Hz,H-8); 6.86(d,1H,J=8.08Hz,H-7); 5.13(dd, J=10.44;7.94 Hz, H-5); 4.41 (bs,1H,H-4); 3.72(d,1H, J=2.07 Hz, H-4); 3.04(bs,6H, NCH₃); 2.90(dd,1H,J=7.94;2.07 Hz, H-4a); 2.70(dq, 1H, J=12.31; 6.56 Hz, H-6); 2.61(dd,1H, J=12.31;10.44 Hz, H-5a); 2.2(m, 6H, J=7.48 Hz, Acetyl); 1.44(bs, 9H, C(CH₃)₃); 1.29(d, 3H, J=6.56 Hz, CH₃); 1.20(t, 3H, J=7.48 Hz, C-CH₃).

### Examples 14

### [4S-(4alpha,12aalpha)]-9-(tert-butyl)-4-(dimethylamino)-5-cyclobutylcarbonyloxy-1,4,4a,5,5a,6,11,12a-octahydro-3,10,12,12a-tetrahydroxy-6-methyl-1,1-dioxo-2-naphthacenecarboxamide (9-t-hutyl-6-deoxy-5-cyclobutylcarbonyloxytetracycline)

To a polypropylene tube containing 0.1 g of doxycycline is added 2 ml of cyclobutanecarboxylic acid in excess. The solution is stirred and cooled in dry ice for 10 minutes followed by careful addition of 15 ml of anhydrous hydrofluoric acid. After 24 hours, the acid is evaporated off under a slow, steady stream if nitrogen to give the 5-ester as a yellow glass. The ester is used without further purification to prepare the title compound according to the procedure in Example 11. Thus, 0.1 g of ester is dissolved in 2 ml of methanesulfonic acid and 1 ml of tert butyl alcohol is added. The reaction is stirred at room temperature and under ambient atmosphere for 18 hours, then poured over ice and extracted with chloroform. The extract is dried and the residue subjected to preparative HPLC to afford the desired product as a yellow glass.

MS(FAB): m/z 583 (M+H).
¹H NMR (CD₃OD): d 7.43 (d, 1H,J=8.08Hz.H-8); 6.84(d,1H.J=8.08Hz,H-7); 5.09(dd, J=10.44; 7.94 Hz, H-5); 4.39(bs,1H,H-4); 3.80(d, 1H, J=2.07 Hz. H-4); 2.98 (bs,6H, NCH₃); 2.91 (dd, 1H,J=7.94;2.07 Hz, H-4a); 2.70(dq, 1H, J=12.31 ; 6.56 Hz, H-6); 2.60(dd,1H, J=12.31;10.44 Hz, H-5a); 2.6-2.7(m, 6H, J=7.48 Hz. CH₂-C); 1.44(bs, 9H. C(CH₃)₃); 1.29(d, 3H, J=6.56 Hz, CH₃); 1.20(t, 3H, J=7.48 Hz, C-CH₃).

### Example 15

General procedure for preparation of 9-alkyl substituted doxycycline derivatives: To a solution of 0.1 g of doxycycline hydrochloride in 1 ml of methanesulfonic acid and 10 drops of hexametaphosphoric acid (HMPA) was added excess corresponding tertiary alcohol. The reaction mixture was stirred over night at room temperature, then ice water was added. The mixture was titrated with dilute NaOH solution to adjust the pH of the solution to 4-5, and extracted with ethyl acetate. The organic extract was separated by preparative HPLC to afford the desired product as a yellow solid.

### [4S-(4alpha, 12aalpha)]-9-[1'-(1'-methyl)cyclohexyl]-4-(dimethylamino)-1,4,4a,5,5a,6,11,12a-octahydro-3,5,10,12,12a-pentahydroxy-6-methyl-1,11-dioxo-2-naphthacenecarboxamide (9-[1'-(1'-methyl)cyclohexyl]-6-deoxy-5-hdroxytetracycline)

MS(FAB): m/z 541 (M+H).

¹H NMR (CD₃OD): d 7.55(d, 1H, J=8.16Hz, H-8); 6.93(d, 1H, J=8.16Hz, H-7); 4.45(bs, 1H, H-4); 3.58(dd, 1H, J=11.42; 8.35Hz, H-5); 2.99, 2.97(each s, each 3H, NMe₂); 2.83(d, 1H, J=11.61Hz, H-4a); 2.75(m, 1H, H-6); 2.60(m, 1H, H-5a); 2.38, 2.06(each t, each 2H, J=8.10Hz, CH₂-2' and CH₂-6'); 1.55(d, 3H, J=6.51 Hz. CH₃-C6): 1.70-1.51 (m, 6H, CH₂-3', CH₂-4', and CH₂-5'); 1.49(s, 3H, CH₃-Cl').

### Example 16

### [4S-(4alpha, 12aalpha)]-9-[1'-(1'-methyl)cyclopentyl]-4-(dimethylamino)-1,4,4a,5,5a,6,11,12a-octahydro-3,5,10,12,12a-pentahydroxy-6-methyl-1,11-dioxo-2-naphthacenecarboxamide (9-[1'-(1'-methyl)cyclopentyl]-6-deoxy-5-hydroxytetracycline)

MS(FAB): m/z 527 (M+H).

¹H NMR (CD₃OD): d 7.44(d, 1H, J=7.67Hz, H-8); 6.83(d, 1H, J=7.67Hz, H-7); 4.46(bs, 1H, H-4); 3.54(dd, 1H, J=11.42; 8.35Hz, H-5); 2.99, 2.91(each s, each 3H. NMe₂); 2.80(d, 1H, J=11.31Hz, H-4a); 2.66(m, 1H, H-6); 2.56(dd. 1H, J=11.42, 8.25Hz, H-5a); 1.94(m, 4H, CH₂-2' and CH₂-5'); 1.74(m, 4H, CH₂-3' and CH₂-4'); 1.50(d, 3H, J=6.51Hz, CH₃-C6); 1.29(bs, 3H, CH₃-C1').

### Example 17

### [4S-(4alpha,12aalpha)]-9-[1'-(1'-methyl)cyclobutyl]-4-(dimethylamino)-1,4,4a,5,5a,6,11,12a-octahydro-3,5,10,12,12a-pentahydroxy-6-methyl-1,11-dioxo-2-naphthacenecarboxamide (9-[1'-(1'-methyl)cyclobutyl-6-deoxy-5-hydroxytetracycline)

Methylenecyclobutane was used and the reaction time was decreased to 5 hrs.

MS(FAB): m/z 513 (M+H).

¹H NMR (CD₃OD): d 7.23(d, 1H, J=7.71Hz, H-8); 6.87(d, 1H, J=7.71 Hz, H-7); 4.46(bs, 1H, H-4); 3.54(dd, 1H, J=11.42; 8.35Hz, H-5); 2.98, 2.92(each s, each 3H, NMe₂); 2.81 (d, 1H, J=11.13Hz, H-4a); 2.72(m, 1H, H-6); 2.59(dd, 1H, J=11.42, 8.25Hz, H-5a); 2.40(m, 2H, CH₂-3'); 2.13(m, 4H, CH₂-2' and CH₂-4'); 1.53(bs, 3H, CH₃-C1'); 1.51 (d, 3H, J=6.51 Hz, CH₃-C₆).

### Example 18

### [4S-(4alpha, 12aalpha)]-9-[2'-(2'-methyl)pentyl]-4-(dimethylamino)-1,4,4a,5,5a,6,11,12a-octahydro-3,5,10,12,12a-pentahydroxy-6-methyl-1,11-dioxo-2-naphthacenecarboxamide (9-[2'-(2'-methyl)pentyl]-6-deoxy-5-hydroxytetracycline)

The reaction was carried out without HMPA.

MS(FAB): m/z 529 (M+H).

¹H NMR (CD₃OD): d 7.41 (d, 1H, J=8.11Hz, H-8); 6.85(d, 1H, J=8.11Hz, H-7); 3.96(bs, 1H, H-4); 3.64(dd, 1H, J=11.42; 8.35Hz, H-5); 2.78(bs, 6H, NMe₂); 2.73(d, 1H, J=11.45Hz, H-4a); 2.51(m, 2H, H-6 and H-5a); 1.86(t, 2H, J=8.22Hz, CH₂-3'); 1.51(d, 3H, J=6.78 Hz, CH₃-C6); 1.38(m, 2H, CH₂-4'); 1.36, 1.28(each s, each 3H, CH₃-1' and CH₃-C2'); 0.82(t, 3H, J=7.17Hz, CH₃-5').

### Example 19

### [4S-(4alpha,12aalpha)]-9-[4'-(1'-bromo-4'-methyl)pentyl]-4-(dimethylamino)-1,4,4a,55a,6,11,12a-octahydro-3,5,10,12,12a-pentahydroxy-6-methyl-1,11-dioxo-2-naphthacenecarboxamide (9-[4'-(1'-bromo-4'-methyl)pentyl]-6-deoxy-5-hydroxytetracycline)

The reaction was carried out with and without HMPA.

MS(FAB): m/z 607 (M+H) and 609 (M+H).

¹H NMR (CD₃OD): d 7.42(d, 1H, J=8.00Hz, H-8); 6.87(d, 1H, J=8.00Hz, H-7); 3.98(bs, 1H, H-4); 3.67(dd, 1H, J=11.42; 8.35Hz, H-5); 3.54(t, 2H, J=7.20Hz, CH₂-1'); 2.79(bs, 6H, NMe₂); 2.74(d, 1H, J=11.45Hz, H-4a); 2.50(m, 2H, H-6 and H-5a); 2.03(m, 2H, CH₂-2'); 1.51 (d, 3H, J=6.71 Hz, CH₃-C6); 1.41 (m, 2H, CH₂-3'); 1.38(s, 6H, CH₃-5' and CH₃-C4').

### Example 20

### [4S-(4alpha,12aalpha)]-9-[4'-(1'-dimethylamino-4'-methyl)pentyl]-4-(dimethylamino)-1,4,4a,5,5a,6,11,12a-octahydro-3,5,10,12,12a-pentahydroxy-6-methyl-1,11-dioxo-2-naphthacenecarboxamide (9-[4'-(1'-dimethylamino-4'-methyl)pentyl]-6-deoxy-5-hydroxytetracycline)

To a solution of 50 mg of the product from example 10 in 1 ml of 1-ethyl-2-pyrrolidinone was added 3 equivalents of dimethylamine (2.0 M solution in methanol). After the mixture was stirred for 3 hrs at room temperature under N₂. it was added dropwise to 50 ml of diethyl ether. The resulting yellow solid was collected and purified by preparative HPLC to give the desired product as a yellow solid.

MS(FAB): m/z 572 (M+H).
¹H NMR (CD₃OD): d 7.51 (d, 1H, J=7.95Hz, H-8); 6.92(d, 1H, J=7.95Hz, H-7); 4.41 (bs, 1H, H-4); 3.65(m, 1H, H-5); 3.72(t, 2H, J=6.62Hz, CH₂-1'); 2.94(bs, 6H, NMe₂-4); 2.78(bs, 6H, NMe₂-1'); 1.53(d, 3H, J=6.71 Hz, CH₃-C6); 1.38(s, 6H, CH₃-5' and CH₃-C4').

### Example 21

### [4S-(4alpha,12a-alpha)]-9-[4'-(1'-pyrrolidinyl-4'-methy/)pentyl]-4-(dimethylamino)-1,4,4a,5,5a,6,11,12a-octahydro-3,5,10,12,12a-pentahydroxy-6-methyl-1,11-dioxo-2-naphthacenecarboxamide (9-[4'-(1'-pyrrolidinyl-4'-methyl)pentyl]-6-deoxy-5-hydroxytetracycline)

To a solution of 50 mg of the product from example 10 in 1 ml of 1-methyl-2-pyrrolidinone was added 3 equivalents of pyrrolidine. After the mixture was stirred for 3hrs at room temperature under N₂, it was added dropwise to 50 ml of cold diethyl ether. The resulting yellow solid was collected and purified by preparative HPLC to give the desired product as a yellow solid.

MS(FAB): m/z 598 (M+H).

### Example 22

### [4S-(4alpha,12aalpha)]-9-[4'-(1'-cyano-4'-methyl)pentyl]-4-(dimethylamino)-1,4,4a,5,5a,6,11,12a-octahydro-3,5,10,12,12a-pentahydroxy-6-methyl-1,11-dioxo-2-naphthacenecarboxamide (9-[4'-(1'-cyano-4'-methyl)pentyl]-6-deoxy-5-hydroxytetracycline)

To a solution of 50 mg of the product from example 10 in 1 ml of DMSO was added 3 equivalents of sodium cyanide. After the mixture was stirred for 3 hrs at room temperature under N₂, 5 ml of methanol was added and purified by preparative HPLC to give the desired product as a brown yellow solid.

MS(FAB): m/z 554 (M+H).
¹H NMR (CD₃OD): d 7.47(d, 1H, J=8.14Hz, H-8); 6.90(d, 1H, J=8.14Hz, H-7); 4.43(bs, 1H, H-4); 3.54(m, 1H, H-5); 2.98, 2.91 (each s, each 3H, NMe₂); 2.82(d, 1H, J=11.45Hz. H-4a); 2.69(dq, 1H, J=12.23, 6.70Hz, H-6); 2.55(dd, 1H, J=12.23, 8.20Hz, H-5a); 2.31(t, 2H, J=6.95Hz, CH₂-1'); 2.05(m, 2H, CH₂-2'); 1.53(d, 3H, J=6.70 Hz, CH₃-C6); 1.50(m, 2H, CH₂-3'); 1.41(s, 6H, CH₃-5' and CH₃-C4').

### Example 23

### [4S-(4alpha,12a-alpha)]-9-[4'-(1'-nitro-4'-methyl)pentyl]-4-(dimethylamino)-1,4,4a,5,5a,6,11,12a-octahydro-3,5,10,12,12a-pentahydroxy-6-methyl-1,11-dioxo-2-naphthacenecarboxamide (9-[4'-(1'-nitro-4'-methyl)pentyl]-6-deoxy-5-hydroxytetracycline)

To a solution of 50 mg of the product from example 10 in 1 ml of DMSO was added 3 equivalents of sodium nitrite. After the mixture was stirred for 3 hrs at room temperature under N₂, 5 ml of methanol was added and purified by preparative HPLC to give the desired product as a brown yellow solid.

MS(FAB): m/z 574 (M+H).

¹H NMR (CD₃OD): d 7.50(d, 1H, J=7.19Hz, H-8); 6.92(d, 1H, J=7.19Hz. H-7); 4.46(bs, 1H, H-4); 4.36(t, 2H, J=6.95Hz, CH₂-1'); 3.61(m, 1H, H-5); 2.98(bs, 6H, NMe₂); 2.85-2.77(m, 2H, H-4a and H-6); 2.62(m, 1H, H-5a): 2.00(m, 2H, CH₂-2'); 1.56(d, 3H, J=6.70 Hz, CH₃-C6); 1.49(m, 2H, CH₂-3'); 1.43(s, 6H, CH₃-5' and CH₃-C4').

### Example 24

### [4S-(4alpha,12aalpha)]-9-[4'-(1'-aceloxy-4'-methyl)pentyl]-4-(dimethylamino)-1,4,4a,5,5a,6,11,12a-octahydro-3,5,10,12,12a-pentahydroxy-6-methyl-1,11-dioxo-2-naphthacenecarboxamide (9-[4'-(1'-aceloxy-4'-methyl)pentyl]-6-deoxy-5-hydroxytetracycline)

To a solution of 50 mg of the product from example 10 in 1 ml of HMPA was added 3 equivalents of sodium acetate. After the mixture was stirred for 3hrs at room temperature under N₂, 5 ml of methanol was added and purified by preparative HPLC to give the desired product as a brown yellow solid.

¹H NMR (CD₃OD): d 7.46(d, 1H, J=8.04 Hz, H-8); 6.89(d, 1H, J=8.04 Hz, H-7); 4.43(bs, 1H, H-4); 3.63(m. 1H, H-5); 3.45 (t, 2H, J=6.72Hz; CH₂-1'); 2.98, 2.91(each s, each 3H, NMe₂); 2.78(d. I H. J=11.45Hz, H-4a); 2.72(dq, 1H, J=12.41, 6.79Hz, H-6); 2.58(dd, 1H, J=12.41, 8.20Hz, H-5a); 2.05(m, 2H. CH₂-2'); 1.52(d, 3H, J=6.79 Hz, CH₃-C6); 1.42(m, 2H. CH₂-3'); 1.40(s, 6H, CH₃-5' and CH₃-C4').

### BIOLOGICAL ACTIVITY

### Method for in vitro Evaluation

### (Table 4)

The minimum inhibitory concentration, the lowest concentration of drug that inhibits bacterial growth at 18 hours at their appropriate temperature, is determined by the broth dilution method using L-broth or Muller-Hinton broth. The Muller-Hinton broth was cation-adjusted accordingly and all bacteriological methods were performed as was described by Waitz, J.A., National Commision for Clinical Laboratory Standards Document M7-A2, vol.10, no. 8, pp.13-20, 2nd edition, Villanova, PA (1990). The organisms tested represent gram-positive and gram-negative bacterial species that are susceptible to tetracyclines or are resistant to tetracyclines due to the ability to efflux tetracyclines or which confer resistance by ribosomal protection mechanisms. The clinical strains used are either susceptible to tetracyclines or are resistant to them by either drug efflux or ribosomal protection.

### Legend for Compounds

| Compound | Name |
|---|---|
| Doxycycline | [4S-(4alpha,12aalpha)]-4-(dimethylamino)-1,4,4a,5,5a,6,11,12a-octahydro-3,5,10,12,12a- pentahydroxy-6-methyl-1,11-dioxo-2-naphthacenecarboxamide |
| **A** | [4S-(4alpha,12aalpha)]-9-(tert-butyl)-4-(dimethylamino)-1,4,4a,5,5a,6,11,12a-octahydro-3,5,10,12,12a-pentahydroxy-6-methyl-1,11-dioxo-2-naphthacenecarboxamide |
| | *(9-t-butyl-6-deoxy-5-hydroxytetracycline)* |
| **B** | [4S-(4alpha, 12aalpha)]-9-(tert-butyl)-4-(dimethylamino)-5-propionylcarbonyloxy-1,4,4a,5,5a,6,1,12a-octahydro-3,10,12,12a-tetrahydroxy-6-methyl-1,11-dioxo-2-naphthacenecarboxamide |
| | *(9-t-butyl-6-deoxy-5-propionylcarbonyloxytetracycline)* |
| **C** | [4S-(4alpha,12aalpha)]-9-(tert-butyl)-4-(dimethylamino)-5-acetylcarbonyloxy-1,4,4a,5,5a,6,11,12a-octahydro-3,10,12,12a-tetrahydroxy-6-methyl-1,11-dioxo-2-naphthacenecarboxamide |
| | *(9-t-butyl-6-deoxy-5-acetylcarbonyloxytetracycline)* |
| **D** | [4S-(4alpha,12aalpha)]-9-(tert-butyl)-4-(dimethylamino)-5-cyclobutylcarbonyloxy-1,4,4a,5,5a,6,11,12a-octahydro-3,10,12,12a-tetrahydroxy-6-methyl-1,11-dioxo-2-naphthacenecarboxamide |
| | *(9-t-butyl-6-deoxy-5-cyclobutylcarbonyloxytetracycline)* |
| **E** | [4S-(4alpha,12aalpha)]-9-[1'-(1'-methyl)cyclohexyl]-4-(dimethylamino)-1,4,4a,5,5a,6,11,12a-octahydro-3,5,10,12,12a-pentahydroxy-6-methyl-1,11-dioxo-2-naphthacenecarboxamide |
| | (9-[1'-(1*'-methyl)cyclohexyl]-6-deoxy-5-hydroxytetracycline)* |
| **F** | [4S-(4alpha,12aalpha)]-9-[1'-(1'-methyl)cyclopentyl]-4-(dimethylamino)-1,4,4a,5,5a,6,]1,]2a-octahydro-3,5,10,12,12a-pentahydroxy-6-methyl-1,11-dioxo-2-naphthacenecarboxamide |
| | *(9-[1'-(1'-methyl)cyclopentyl]-6-deoxy-5-hydroxytetracycline)* |
| **G** | [4S-(4alpha,12aalpha)]-9-[1'-(1'-methyl)cyclobutyl]-4-(dimethylamino)-1,4,4a,5,5a,6,11,12a-octahydro-3,5,10,12,12a-pentahydroxy-6-methyl-1,11-dioxo-2-naphthacenecarboxamide |
| | *(9-[1'-(1'-methyl)cyclobutyl]-6-deoxy-5-hydroxytetracycline)* |
| **H** | [4S-(4alpha,12aalpha)]-9-[2'-(2'-methyl)pentyl]-4-(dimethylamino)-1,4,4a,5,5a,6,11,12a-octahydro-3,5,10,12,12a-pentahydroxy-6-methyl-1,11-dioxo-2-naphthacenecarboxamide |
| | (9-[2'-(2'-methyl)pentyl]-6-deoxy-5-hydroxytetracycline) |
| **I** | [4S-(4alpha,12aalpha)]-9-[4'-(1'-bromo-4'-methyl)pentyl]-4-(dimethylamino)-1,4,4a,5,5a,6,11,12a-octahydro-3,5,10,12,12a-pentahydroxy-6-methyl-1,11-dioxo-2-naphthacenecarboxamide |
| | *(9-[4'-(1'-bromo-4'-methyl)pentyl]-6-deoxy-5-hydroxytetracycline)* |
| **J** | [4S-(4alpha,12aalpha)]-9-[4'-(1'-dimethylamino-4'-methyl)pentyl]-4-(dimethylamino)-1,4,4a,5,5a,6,11,12a-octahydro-3,5,10,12,12a-pentahydroxy-6-methyl-1,11-dioxo-2-naphthacenecarboxamide |
| | *(9-[4'-(1'-dimethylamino-4'-methyl)pentyl]-6-deoxy-5-hydroxytetracycline)* |
| **K** | [4S-(4alpha,12a-alpha)]-9-[4'-(1'-pyrrolidinyl-4'-methyl)pentyl]-4-(dimethylamino)-1,4,4a,5,5a,6,11,12a-octahydro-3,5,10,12,12a-pentahydroxy-6-methyl-1,11-dioxo-2-naphthacenecarboxamide |
| | *(9-[4'-(1'-pyrrolidinyl-4'-methyl)pentyl]-6-deoxy-5-hydroxytetracycline)* |
| **L** | [4S-(4alpha,12aalpha)]-9-[4'-(1'-cyano-4'-methyl)pentyl]-4-(dimethylamino)-1,4,4a,5,5a,6,11,12a-octahydro-3,5,10,12,12a-pentahydroxy-6-methyl-1,11-dioxo-2-naphthacenecarboxamide |
| | *(9-[4'-(1'-cyano*-4*'-methyl)pentyl]-6-deoxy-5-hydroxytetracycline)* |
| **M** | [4S-(4alpha,12a-alpha)]-9-[4'-(1'-nitro-4'-methyl)pentyl)-4-(dimethylamino)-1,4,4a,5,5a,6,11,12a-octahydro-3,5,10,12,12a-pentahydroxy-6-methyl-1,11-dioxo-2-naphthacenecarboxamide |
| | *(9-[4'-(1'-nitro*-4*'-methyl)pentyl]-6-deoxy-5-hydroxytetracycline)* |
| **N** | [4S-(4alpha,12aalpha)]-9-[4'-(1'-acetoxy-4'-methyl)pentyl]-4-(dimethylamino)-1,4,4a,5,5a,6,11,12a-octahydro-3,5,10,12,12a-pentahydroxy-6-methyl-1,11-dioxo-2-naphthacenecarboxamide |
| | *(9-[4'-(1'-acetoxy*-4*'-methyl)pentyl]-6-deoxy-5-hydroxytetracycline)* |

**Table 4**

| Antibacterial activity of substituted tetracyclines | | | | | | | | | | | | | | | |
|---|---|---|---|---|---|---|---|---|---|---|---|---|---|---|---|
| | Doxycycline | A | B | C | D | E | F | G | H | I | J | K | L | M | N |
| *E.coli* D31m4* Tc^{r} | 0.78 | 3.12 | 6.25 | 3.12 | 50 | 25 | 0.78 | 6.25 | 50 | 1.56 | 50 | 50 | 6.25 | 12.5 | 3.12 |
| *E.coli D31m4(pHCM1)* Tcr | 12.5 | 3.12 | 6.25 | 3.12 | 50 | 25 | 1.56 | 6.25 | 1.56 | 1.56 | 50 | 50 | 6.25 | 1.56 | 3.12 |
| *S. aureus RN450 Tc^{s}* | 0.195 | 0.78 | 0.39 | 0.39 | 0.78 | 1.56 | 0.39 | 1.56 | 0.08 | 0.39 | 50 | 25 | 1.56 | 3.12 | 1.56 |
| *S. aureus Tc^{r} ATCC12715* | 50 | 3.12 | 1.56 | 0.78 | 3.12 | 6.25 | 0.78 | 3.12 | 0.78 | 0.78 | 50 | 50 | 6.25 | 1.56 | 1.56 |
| *S. aureus* RN4250 Tc^{r} | 25 | 1.56 | 0.78 | 0.39 | 3.12 | 6.25 | 0.39 | 3.12 | 0.39 | 0.08 | 50 | 50 | 3.12 | 0.78 | 1.56 |
| *S. aureus* MRSA5 Tc^{r} | 6.25 | 3.12 | 3.12 | 1.56 | 1.56 | 6.25 | 0.08 | 0.08 | 0.39 | 0.39 | 50 | 6.25 | 6.25 | 0.78 | 1.56 |
| *E. faecalis* Tc^{r} ATCC9790 | 0.39 | 1.56 | 1.56 | 0.78 | 0.78 | 0.39 | 0.78 | 0.78 | 0.78 | 0.39 | 50 | 25 | 3.12 | 0.78 | 1.56 |
| *E. faecalis pMV158* Tc^{r} | 6.25 | 1.56 | 0.39 | 0.39 | 50 | 1.56 | 0.08 | 1.56 | 0.78 | 0.08 | 50 | 25 | 3.12 | 0.78 | 0.78 |
| *E. faecalis* pAM211 Tc^{r} | 12.5 | 3.12 | 0.78 | 0.78 | 1.56 | 6.25 | 0.39 | 3.12 | 0.78 | 0.78 | 50 | 25 | 3.12 | 0.78 | 1.56 |

| | | | | | | | | | | | | | | | |
|---|---|---|---|---|---|---|---|---|---|---|---|---|---|---|---|
| Tc^{s} = tetracycline susceptible Tc^{r} = tetracycline resistant * lipopolysaccharide-deficient mutant | | | | | | | | | | | | | | | |

### Equivalents

Those skilled in the art will recognize, or be able to ascertain using no more than routine experimentation, numerous equivalents to the specific procedures described herein. Such equivalents are considered to be within the scope of this invention and are covered by the following claims.

## Claims

1. A 5,9-substituted tetracycline, of the following Formula I: 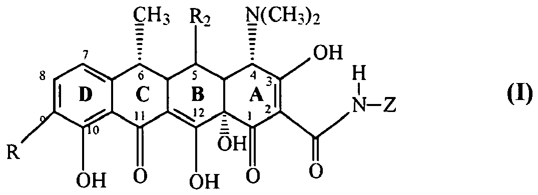
wherein R is alkyl; alkenyl; alkynyl; alkoxy; alkylthio; alkylsulfinyl; alkylsulfonyl; an alkyl group having an amino group attached thereto; or an arylalkyl;
R² is alkanoyl; aroyl; alkaroyl; carbocyclic aryl; heteroaromatic; alkyl; alkenyl; alkynyl; alkoxy; alkylthio; alkylsulfinyl; alkylsulfonyl; an alkyl group having an amino group attached thereto; or an arylalkyl;
Z is hydrogen; alkyl; alkenyl; alkynyl; alkoxy; alkylthio; alkylsulfinyl; alkylsulfonyl; arylalkyl; carbocyclic aryl; an alkyl group having an amino group attached thereto; or heteroalicyclic; or a pharmaceutically acceptable salt thereof.

2. A compound of claim 1 selected from:
5-propionate-9-t-butyl doxycycline;
9-chloro-t-butyl-5-propionate doxycycline;
9-t-butyl-6-alpha-deoxy-5-formyloxy-tetracycline;
9-t-butyl-6-alpha-deoxy-5-acetoxy-tetracycline;
9-t-butyl-6-alpha-deoxy-5-propionyloxy-tetracycline;
9-t-butyl-6-alpha-deoxy-5-phenylcarbonyloxy-tetracycline;
9-t-butyl-6-alpha-deoxy-5-benzylcarbonyloxy-tetracycline;
9-t-butyl-6-alpha-deoxy-5-dimethylaminocarbonyloxy-tetracycline;
9-t-butyl-6-alpha-deoxy-5-cyclopentylcarbonyloxy-tetracycline;
9-t-butyl-6-alpha-deoxy-5-cyclobutylcarbonyloxy-tetracycline;
9-t-butyl-6-alpha-deoxy-5-cyclohexylcarbonyloxy-tetracycline;
9-t-butyl-6-alpha-deoxy-5-cycloheptylcarbonyloxy-tetracycline;
9-[(dimethylamino)-t-butyl]-6-alpha-deoxy-5-formyloxy-tetracycline;
9-[(dimethylamino)-t-butyl]-6-alpha-deoxy-5-acetoxy-tetracycline;
9-[(dimethylamino)-t-butyl]-6-alpha-deoxy-5-propionylcarbonyloxy-tetracycline;
9-[(dimethylamino)-t-butyl]-6-alpha-deoxy-5-phenylcarbonyloxy-tetracycline;
9-[(dimethylamino)-t-butyl]-6-alpha-deoxy-5-benzylcarbonyloxy-tetracycline;
9-[(dimethylamino)-t-butyl]-6-alpha-deoxy-5-dimethylaminocarbonyloxy-tetracycline;
9-[(dimethylamino)-t-butyl]-6-alpha-deoxy-5-cyclopentylcarbonyloxy-tetracycline;
9-[(dimethylamino)-t-butyl]-6-alpha-deoxy-5-cyclobutylcarbonyloxy-tetracycline;
9-[(dimethylamino)-t-butyl]-6-alpha-deoxy-5-cyclohexylcarbonyloxy-tetracycline;
9-[(dimethylamino)-t-butyl]-6-alpha-deoxy-5-cycloheptylcarbonyloxy-tetracycline; and
pharmaceutically acceptable salts thereof.

3. The compound of claim 1 wherein R is alkyl having 1 to 20 carbon atoms; alkenyl having 2 to 20 carbon atoms; alkynyl having 2 to 20 carbon atoms; alkoxy having 1 to 20 carbon atoms; alkylthio having 1 to 20 carbon atoms; alkylsulfinyl having 1 to 20 carbon atoms; alkylsulfonyl having 1 to 20 carbon atoms; an alkyl group having 1 to 20 carbon atoms and an amino group attached thereto; or arylalkyl;
R² is alkyl having 1 to 20 carbon atoms; alkenyl having 2 to 20 carbon atoms; alkynyl having 2 to 20 carbon atoms; alkoxy having 1 to 20 carbon atoms; alkylthio having 1 to 20 carbon atoms; alkylsulfinyl having 1 to 20 carbon atoms; alkylsulfonyl having 1 to 20 carbon atoms; an alkyl group having 1 to 20 carbon atoms and an amino group attached thereto; arylalkyl; alkanoyl having 1 to 20 carbon atoms; aroyl; alkaroyl; carbocyclic aryl; or heteroaromatic; and
Z is hydrogen, alkyl having 1 to 20 carbon atoms; alkenyl having 2 to 20 carbon atoms; alkynyl having 2 to 20 carbon atoms; alkoxy having 1 to 20 carbon atoms; alkylthio having 1 to 20 carbon atoms; alkylsulfinyl having 1 to 20 carbon atoms; alkylsulfonyl having 1 to 20 carbon atoms; an alkyl group having 1 to 20 carbon atoms and an amino group attached thereto; arylalkyl; carbocyclic aryl; or a heteroalicyclic group.

4. The compound of claim 3 wherein Z is hydrogen; and R is alkyl having 1 to 12 carbon atoms; alkenyl having 2 to 12 carbon atoms; alkynyl having 2 to 12 carbon atoms; alkoxy having 1 to 12 carbon atoms; alkylthio having 1 to 12 carbon atoms; alkylsulfinyl having 1 to 12 carbon atoms; alkylsulfonyl having 1 to 12 carbon atoms; an alkyl group having 1 to 12 carbon atoms and an amino group attached thereto; or benzyl.

5. The compound of claim 1, wherein R is alkyl having 1 to 12 carbon atoms; alkenyl having 2 to 12 carbon atoms; alkynyl having 2 to 12 carbon atoms; alkoxy having 1 to 12 carbon atoms; alkylthio having 1 to 12 carbon atoms; alkylsulfinyl having 1 to 12 carbon atoms; alkylsulfonyl having 1 to 12 carbon atoms; an alkyl group having 1 to 12 carbon atoms and an amino group attached thereto; or benzyl;
R² is alkyl having 1 to 12 carbon atoms; alkenyl having 2 to 12 carbon atoms; alkynyl having 2 to 12 carbon atoms; alkoxy having 1 to 12 carbon atoms; alkylthio having 1 to 12 carbon atoms; alkylsulfinyl having 1 to 12 carbon atoms; alkylsulfonyl having 1 to 12 carbon atoms; an alkyl group having 1 to 12 carbon atoms and an amino group attached thereto; benzyl; aroyl; alkaroyl; carbocyclic aryl; or heteroaromatic;
and Z is hydrogen.

6. The compound of claim 1, wherein R is selected from the group consisting of t-butyl; chloro-t-butyl; piperidinoethyl; and (dimethylamino)-t-butyl; and R² is selected from the group consisting of propionate; formyloxy; acetoxy; propionyloxy; phenylcarbonyloxy; benzylcarbonyloxy; acetylcarbonyloxy; propionylcarbonyloxy; phenylcarbonyloxy; benzylcarbonyloxy; dimethylaminocarbonyloxy; cyclopentylcarbonyloxy; cyclobutylcarbonyloxy; cyclohexylcarbonyloxy; and cycloheptylcarbonyloxy; and Z is hydrogen.

7. The compound of claim 1, wherein said compound is selected from the group consisting of
9-t-butyl-6-deoxy-5-propionylcarbonyloxytetracycline,
9-t-butyl-6-deoxy-5-acetylcarbonyloxytetracycline,
9-t-butyl-6-deoxy-5-cyclobutylcarbonyloxytetracycline, and
pharmaceutically acceptable salts thereof.

8. A compound of the following Formula II: 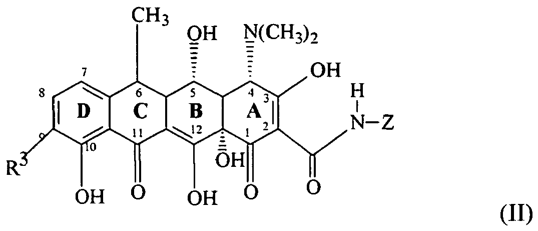
wherein R³ is alkyl; alkenyl; alkynyl; alkoxy; alkylthio; alkylsulfinyl; alkylsulfonyl; alkylaryl;
Z is hydrogen, alkyl; alkenyl; alkynyl; alkoxy; alkylthio; alkylsulfinyl; alkylsulfonyl; arylalkyl; carbocyclic aryl; heteroalicyclic; an alkyl group having an amino group attached thereto; or heteroaromatic group;
or a pharmaceutically acceptable salt thereof.

9. The compound of claim 8 wherein R³ is alkyl having 1 to 20 carbon atoms; alkenyl having 2 to 20 carbon atoms; alkynyl having 2 to 20 carbon atoms; alkoxy having 1 to 20 carbon atoms; alkylthio having 1 to 20 carbon atoms; alkylsulfinyl having 1 to 20 carbon atoms; alkylsulfonyl having 1 to 20 carbon atoms; an alkyl group having 1 to 20 carbon atoms and an amino group attached thereto; or arylalkyl; and
Z is hydrogen, alkyl having 1 to 20 carbon atoms; alkenyl having 2 to 20 carbon atoms; alkynyl having 2 to 20 carbon atoms; alkoxy having 1 to 20 carbon atoms; alkylthio having 1 to 20 carbon atoms; alkylsulfinyl having 1 to 20 carbon atoms; alkylsulfonyl having 1 to 20 carbon atoms; an alkyl group having 1 to 20 carbon atoms and an amino group attached thereto; arylalkyl; carbocyclic aryl; or a heteroalicyclic group.

10. The compound of claim 8, wherein said compound is selected from the group consisting of
9-t-butyl-6-deoxy-5-hydroxytetracycline,
9-[1'-(1'-methyl) cyclohexyl]-6-deoxy-5-hydroxytetracycline,
9-[1'-(1'-methyl) cyclopentyl]-6-deoxy-5-hydroxytetracycline,
9-[1'-(1'-methyl) cyclobutyl]-6-deoxy-5-hydroxytetracycline,
9-[2'-(2'-methyl)pentyl]-6-deoxy-5-hydroxytetracycline,
9-[4'-(1'-bromo-4'-methyl) pentyl]-6-deoxy-5-hydroxytetracycline,
9-[4'-(1'-dimethylamino-4'-methyl) pentyl]-6-deoxy-5-hydroxytetracycline,
9-[4'-(1'-pyrrolidinyl-4'-methyl) pentyl]-6-deoxy-5-hydroxytetracycline,
9-[4'-(1'-cyano-4'-methyl) pentyl]-6-deoxy-5-hydroxytetracycline,
9-[4'-(1'-nitro-4'-methyl) pentyl]-6-deoxy-5-hydroxytetracycline,
9-[4'-(1'-acetoxy-4'- methyl) pentyl]-6-deoxy-5-hydroxytetracycline,
9-(chloro-t-butyl)-6-deoxy-oxytetracycline,
9-[(dimethylamino)-t-butyl]-6-deoxy-oxytetracycline,
9-(amino)-t-butyl-6-deoxy-oxytetracycline,
9-[(piperidino)-t-butyl]-6-deoxy-oxytetracycline,
9-[(diethylamino)-t-butyl]-6-deoxy-oxytetracycline,
9-[(dipropylamino)-t-butyl]-6-deoxy-oxytetracycline,
and pharmaceutically acceptable salts thereof.

11. The compound of claim 9, wherein R³ is alkyl having 1 to 12 carbon atoms; alkenyl having 2 to 12 carbon atoms; alkynyl having 2 to 12 carbon atoms; alkoxy having 1 to 12 carbon atoms; alkylthio having 1 to 12 carbon atoms; alkylsulfinyl having 1 to 12 carbon atoms; alkylsulfonyl having 1 to 12 carbon atoms; an alkyl group having 1 to 12 carbon atoms and an amino group attached thereto; or benzyl; and
Z is hydrogen.

12. The compound of claim 9, wherein R³ is selected from the group consisting of t-butyl; chloro-t-butyl; (dimethylamino)-t-butyl; methylcyclohexyl; methylcyclobutyl; methylpentyl; bromomethylpentyl; nitromethylpentyl; and acetoxymethylpentyl.

13. A pharmaceutical composition comprising a compound as defined in any of the claims 1-12.

14. A compound of any of the claims 1-12 for use as a medicament.

15. A compound of any one of claims 1-12 for use in treatment against a targeted microorganism.

16. A compound of any one of claims 1-12 for use in treatment against a bacterium.

17. A method for converting tetracycline resistant bacteria into tetracycline sensitive bacteria, comprising
a) contacting the resistant bacteria with a predetermined quantity of a compound of any one of claims 1-12, and
b) concomitantly administering to the bacteria a predetermined quantity of a tetracycline-type compound that is different than the compound of step a).

## Patentansprüche

1. 5,9-substituiertes Tetracyclin der folgenden Formel I: 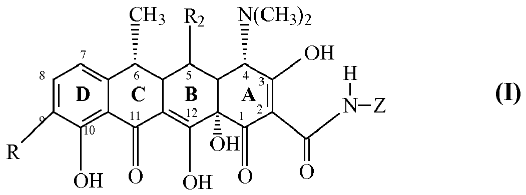
wobei R Alkyl; Alkenyl; Alkinyl; Alkoxy; Alkylthio; Alkylsulfinyl; Alkylsulfonyl; eine Alkylgruppe mit einer daran gebundenen Aminogruppe; oder ein Arylalkyl ist;
R² Alkanoyl; Aroyl; Alkaroyl; ein carbocyclisches Aryl; eine heteroaromatische Gruppe; Alkyl; Alkenyl; Alkinyl; Alkoxy; Alkylthio; Alkylsulfinyl; Alkylsulfonyl; eine Alkylgruppe mit einer daran gebundenen Aminogruppe; oder ein Arylalkyl ist;
Z Wasserstoff; Alkyl; Alkenyl; Alkinyl; Alkoxy; Alkylthio; Alkylsulfinyl; Alkylsulfonyl; Arylalkyl; ein carbocyclisches Aryl; eine Alkylgruppe mit einer daran gebundenen Aminogruppe; oder eine heteroalicyclische Gruppe; oder ein pharmazeutisch unbedenkliches Salz davon ist.

2. Verbindung nach Anspruch 1, ausgewählt aus:
5-Propionat-9-t-butyldoxycyclin;
9-Chlor-t-butyl-5-propionatdoxycyclin;
9-t-Butyl-6-alpha-deoxy-5-formyloxytetracyclin;
9-t-Butyl-6-alpha-deoxy-5-acetoxytetracyclin;
9-t-Butyl-6-alpha-deoxy-5-propionyloxytetracyclin;
9-t-Butyl-6-alpha-deoxy-5-phenylcarbonyloxytetracyclin;
9-t-Butyl-6-alpha-deoxy-5-benzylcarbonyloxytetracyclin;
9-t-Butyl-6-alpha-deoxy-5-dimethylaminocarbonyloxytetracyclin;
9-t-Butyl-6-alpha-deoxy-5-cyclopentylcarbonyloxytetracyclin;
9-t-Butyl-6-alpha-deoxy-5-cyclobutylcarbonyloxytetracyclin;
9-t-Butyl-6-alpha-deoxy-5-cyclohexylcarbonyloxytetracyclin;
9-t-Butyl-6-alpha-deoxy-5-cycloheptylcarbonyloxytetracyclin;
9-[(Dimethylamino)-t-butyl]-6-alpha-deoxy-5-formyloxytetracyclin;
9-[(Dimethylamino)-t-butyl]-6-alpha-deoxy-5-acetoxytetracyclin;
9-[(Dimethylamino)-t-butyl]-6-alpha-deoxy-5-propionylcarbonyloxytetracyclin;
9-[(Dimethylamino)-t-butyl]-6-alpha-deoxy-5-phenylcarbonyloxytetracyclin;
9-[(Dimethylamino)-t-butyl]-6-alpha-deoxy-5-benzylcarbonyloxytetracyclin;
9-[(Dimethylamino)-t-butyl]-6-alpha-deoxy-5-dimethylaminocarbonyloxytetracyclin;
9-[(Dimethylamino)-t-butyl]-6-alpha-deoxy-5-cyclopentylcarbonyloxytetracyclin;
9-[(Dimethylamino)-t-butyl]-6-alpha-deoxy-5-cyclobutylcarbonyloxytetracyclin;
9-[(Dimethylamino)-t-butyl]-6-alpha-deoxy-5-cyclohexylcarbonyloxytetracyclin;
9-[(Dimethylamino)-t-butyl]-6-alpha-deoxy-5-cycloheptylcarbonyloxytetracyclin; und
pharmazeutisch unbedenklichen Salzen davon.

3. Verbindung nach Anspruch 1, wobei R Alkyl mit 1 bis 20 Kohlenstoffatomen; Alkenyl mit 2 bis 20 Kohlenstoffatomen; Alkinyl mit 2 bis 20 Kohlenstoffatomen; Alkoxy mit 1 bis 20 Kohlenstoffatomen; Alkylthio mit 1 bis 20 Kohlenstoffatomen; Alkylsulfinyl mit 1 bis 20 Kohlenstoffatomen; Alkylsulfonyl mit 1 bis 20 Kohlenstoffatomen; eine Alkylgruppe mit 1 bis 20 Kohlenstoffatomen und mit einer daran gebundenen Aminogruppe; oder Arylalkyl ist;
R² Alkyl mit 1 bis 20 Kohlenstoffatomen; Alkenyl mit 2 bis 20 Kohlenstoffatomen; Alkinyl mit 2 bis 20 Kohlenstoffatomen; Alkoxy mit 1 bis 20 Kohlenstoffatomen; Alkylthio mit 1 bis 20 Kohlenstoffatomen; Alkylsulfinyl mit 1 bis 20 Kohlenstoffatomen; Alkylsulfonyl mit 1 bis 20 Kohlenstoffatomen; eine Alkylgruppe mit 1 bis 20 Kohlenstoffatomen und mit einer daran gebundenen Aminogruppe; Arylalkyl; Alkanoyl mit 1 bis 20 Kohlenstoffatomen; Aroyl; Alkaroyl; ein carbocyclisches Aryl; oder eine heteroaromatische Gruppe ist; und
Z Wasserstoff, Alkyl mit 1 bis 20 Kohlenstoffatomen; Alkenyl mit 2 bis 20 Kohlenstoffatomen; Alkinyl mit 2 bis 20 Kohlenstoffatomen; Alkoxy mit 1 bis 20 Kohlenstoffatomen; Alkylthio mit 1 bis 20 Kohlenstoffatomen; Alkylsulfinyl mit 1 bis 20 Kohlenstoffatomen; Alkylsulfonyl mit 1 bis 20 Kohlenstoffatomen; eine Alkylgruppe mit 1 bis 20 Kohlenstoffatomen und mit einer daran gebundenen Aminogruppe; Arylalkyl; ein carbocyclisches Aryl; oder eine heteroalicyclische Gruppe ist.

4. Verbindung nach Anspruch 3, wobei Z Wasserstoff ist; und R Alkyl mit 1 bis 12 Kohlenstoffatomen; Alkenyl mit 2 bis 12 Kohlenstoffatomen; Alkinyl mit 2 bis 12 Kohlenstoffatomen; Alkoxy mit 1 bis 12 Kohlenstoffatomen; Alkylthio mit 1 bis 12 Kohlenstoffatomen; Alkylsulfinyl mit 1 bis 12 Kohlenstoffatomen; Alkylsulfonyl mit 1 bis 12 Kohlenstoffatomen; eine Alkylgruppe mit 1 bis 12 Kohlenstoffatomen und mit einer daran gebundenen Aminogruppe; oder Benzyl ist.

5. Verbindung nach Anspruch 1, wobei R Alkyl mit 1 bis 12 Kohlenstoffatomen; Alkenyl mit 2 bis 12 Kohlenstoffatomen; Alkinyl mit 2 bis 12 Kohlenstoffatomen; Alkoxy mit 1 bis 12 Kohlenstoffatomen; Alkylthio mit 1 bis 12 Kohlenstoffatomen; Alkylsulfinyl mit 1 bis 12 Kohlenstoffatomen; Alkylsulfonyl mit 1 bis 12 Kohlenstoffatomen; eine Alkylgruppe mit 1 bis 12 Kohlenstoffatomen und mit einer daran gebundenen Aminogruppe; oder Benzyl ist;
R² Alkyl mit 1 bis 12 Kohlenstoffatomen; Alkenyl mit 2 bis 12 Kohlenstoffatomen; Alkinyl mit 2 bis 12 Kohlenstoffatomen; Alkoxy mit 1 bis 12 Kohlenstoffatomen; Alkylthio mit 1 bis 12 Kohlenstoffatomen; Alkylsulfinyl mit 1 bis 12 Kohlenstoffatomen; Alkylsulfonyl mit 1 bis 12 Kohlenstoffatomen; eine Alkylgruppe mit 1 bis 12 Kohlenstoffatomen und mit einer daran gebundenen Aminogruppe; Benzyl; Aroyl; Alkaroyl; ein carbocyclisches Aryl; oder eine heteroaromatische Gruppe ist;
und Z Wasserstoff ist.

6. Verbindung nach Anspruch 1, wobei R ausgewählt ist aus der Gruppe, bestehend aus t-Butyl; Chlor-t-butyl; Piperidinoethyl; und (Dimethylamino)-t-butyl; und R² ausgewählt ist aus der Gruppe, bestehend aus Propionat; Formyloxy; Acetoxy; Propionyloxy; Phenylcarbonyloxy; Benzylcarbonyloxy; Acetylcarbonyloxy; Propionylcarbonyloxy; Phenylcarbonyloxy; Benzylcarbonyloxy; Dimethylaminocarbonyloxy; Cyclopentylcarbonyloxy; Cyclobutylcarbonyloxy; Cyclohexylcarbonyloxy; und Cycloheptylcarbonyloxy; und Z ist Wasserstoff.

7. Verbindung nach Anspruch 1, wobei die Verbindung ausgewählt ist aus der Gruppe, bestehend aus
9-t-Butyl-6-deoxy-5-propionylcarbonyloxytetracyclin;
9-t-Butyl-6-deoxy-5-acetylcarbonyloxytetracyclin;
9-t-Butyl-6-deoxy-5-cyclobutylcarbonyloxytetracyclin; und
pharmazeutisch unbedenklichen Salzen davon.

8. Verbindung der folgenden Formel II: 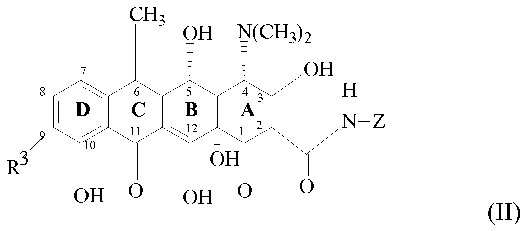
wobei R³ Alkyl; Alkenyl; Alkinyl; Alkoxy; Alkylthio; Alkylsulfinyl; Alkylsulfonyl; Arylalkyl ist;
Z Wasserstoff, Alkyl; Alkenyl; Alkinyl; Alkoxy; Alkylthio; Alkylsulfinyl; Alkylsulfonyl; Arylalkyl; ein carbocyclisches Aryl; eine heteroalicyclische Gruppe; eine Alkylgruppe mit einer daran gebundenen Aminogruppe; oder eine heteroaromatische Gruppe ist;
oder ein pharmazeutisch unbedenkliches Salz davon.

9. Verbindung nach Anspruch 8, wobei R³ Alkyl mit 1 bis 20 Kohlenstoffatomen; Alkenyl mit 2 bis 20 Kohlenstoffatomen; Alkinyl mit 2 bis 20 Kohlenstoffatomen; Alkoxy mit 1 bis 20 Kohlenstoffatomen; Alkylthio mit 1 bis 20 Kohlenstoffatomen; Alkylsulfinyl mit 1 bis 20 Kohlenstoffatomen; Alkylsulfonyl mit 1 bis 20 Kohlenstoffatomen; eine Alkylgruppe mit 1 bis 20 Kohlenstoffatomen und mit einer daran gebundenen Aminogruppe; oder Arylalkyl ist; und
Z Wasserstoff, Alkyl mit 1 bis 20 Kohlenstoffatomen; Alkenyl mit 2 bis 20 Kohlenstoffatomen; Alkinyl mit 2 bis 20 Kohlenstoffatomen; Alkoxy mit 1 bis 20 Kohlenstoffatomen; Alkylthio mit 1 bis 20 Kohlenstoffatomen; Alkylsulfinyl mit 1 bis 20 Kohlenstoffatomen; Alkylsulfonyl mit 1 bis 20 Kohlenstoffatomen; eine Alkylgruppe mit 1 bis 20 Kohlenstoffatomen und mit einer daran gebundenen Aminogruppe; Arylalkyl; ein carbocyclisches Aryl; oder eine heteroalicyclische Gruppe ist.

10. Verbindung nach Anspruch 8, wobei die Verbindung ausgewählt ist aus der Gruppe, bestehend aus
9-t-Butyl-6-deoxy-5-hydroxytetracyclin;
9-[1'-(1'-Methyl)cyclohexyl]-6-deoxy-5-hydroxytetracyclin,
9-[1'-(1'-Methyl)cyclopentyl]-6-deoxy-5-hydroxytetracyclin,
9-[1'-(1'-Methyl)cyclobutyl]-6-deoxy-5-hydroxytetracyclin,
9-[2'-(2'-Methyl)pentyl]-6-deoxy-5-hydroxytetracyclin,
9-[4'-(1'-Brom-4'-methyl)pentyl]-6-deoxy-5-hydroxytetracyclin,
9-[4'-(1'-Dimethylamino-4'-methyl)pentyl]-6-deoxy-5-hydroxytetracyclin,
9-[4'-(1'-Pyrrolidinyl-4'-methyl)pentyl]-6-deoxy-5-hydroxytetracyclin,
9-[4'-(1'-Cyano-4'-methyl)pentyl]-6-deoxy-5-hydroxytetracyclin,
9-[4'-(1'-Nitro-4'-methyl)pentyl]-6-deoxy-5-hydroxytetracyclin,
9-[4'-(1'-Acetoxy-4'-methyl)pentyl]-6-deoxy-5-hydroxytetracyclin,
9-(Chlor-t-butyl)-6-deoxy-oxytetracyclin,
9-[(Dimethylamino)-t-butyl]-6-deoxy-oxytetracyclin,
9-(Amino)-t-butyl-6-deoxy-oxytetracyclin,
9-[(Piperidino)-t-butyl]-6-deoxy-oxytetracyclin,
9-[(Diethylamino)-t-butyl]-6-deoxy-oxytetracyclin,
9-[(Dipropylamino)-t-butyl]-6-deoxy-oxytetracyclin,
und pharmazeutisch unbedenklichen Salzen davon.

11. Verbindung nach Anspruch 9, wobei R³ Alkyl mit 1 bis 12 Kohlenstoffatomen; Alkenyl mit 2 bis 12 Kohlenstoffatomen; Alkinyl mit 2 bis 12 Kohlenstoffatomen; Alkoxy mit 1 bis 12 Kohlenstoffatomen; Alkylthio mit 1 bis 12 Kohlenstoffatomen; Alkylsulfinyl mit 1 bis 12 Kohlenstoffatomen; Alkylsulfonyl mit 1 bis 12 Kohlenstoffatomen; eine Alkylgruppe mit 9 bis 12 Kohlenstoffatomen und mit einer daran gebundenen Aminogruppe; oder Benzyl ist und
Z Wasserstoff ist.

12. Verbindung nach Anspruch 9, wobei R³ ausgewählt ist aus der Gruppe, bestehend aus t-Butyl; Chlor-t-butyl; (Dimethylamino)-t-butyl; Methylcyclohexyl; Methylcyclobutyl; Methylpentyl; Brommethylpentyl; Nitromethylpentyl; und Acetoxymethylpentyl.

13. Pharmazeutische Zusammensetzung, umfassend eine Verbindung nach einem der Ansprüche 1-12.

14. Verbindung nach einem der Ansprüche 1-12 zur Verwendung als Arzneimittel.

15. Verbindung nach einem der Ansprüche 1-12 zur Verwendung bei einer gegen einen Zielmikroorganismus gerichteten Behandlung.

16. Verbindung nach einem der Ansprüche 1-12 zur Verwendung bei einer gegen eine Bakterie gerichteten Behandlung.

17. Verfahren zur Umwandung tetracylinresistenter Bakterien in tetracyclinempfindliche Bakterien, umfassend
a) Kontaktieren der resistenten Bakterie mit einer vorbestimmten Menge einer Verbindung nach einem der Ansprüche 1-12, und
b) gleichzeitiges Verabreichen einer vorbestimmten Menge einer Verbindung vom Tetracyclintyp, die sich von der Verbindung in Schritt a) unterscheidet, an die Bakterie.

## Revendications

1. Tétracycline substituée en 5,9, de formule I suivante : 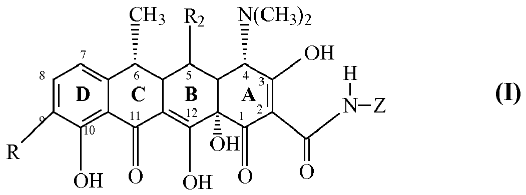
dans laquelle R est un alkyle, un alcényle, un alcynyle, un alkoxy, un alkylthio, un alkylsulfinyle, un alkylsulfonyle, un groupe alkyle auquel est lié un groupe aminé, ou un arylalkyle ;
R² est un alcanoyle, un aroyle, un alcaroyle, un aryle carbocyclique, un hétéroaromatique, un alkyle, un alcényle, un alcynyle, un alkoxy, un alkylthio, un alkylsulfinyle, un alkylsulfonyle, un groupe alkyle auquel est lié un groupe aminé, ou un arylalkyle ;
Z est un hydrogène, un alkyle, un alcényle, un alcynyle, un alkoxy, un alkylthio, un alkylsulfinyle, un alkylsulfonyle, un arylalkyle, un aryle carbocyclique, un groupe alkyle auquel est lié un groupe aminé, ou un hétéroalicyclique, ou un sel pharmaceutiquement acceptable de ceux-ci.

2. Composé de la revendication 1 choisi parmi :
5-propionate-9-t-butyl doxycycline,
9-chloro-t-butyl-5-propionate doxycycline,
9-t-butyl-6-alpha-désoxy-5-formyloxy-tétracycline,
9-t-butyl-6-alpha-désoxy-5-acétoxy-tétracycline,
9-t-butyl-6-alpha-désoxy-5-propionyloxy-tétracycline,
9-t-butyl-6-alpha-désoxy-5-phénylcarbonyloxy-tétracycline,
9-t-butyl-6-alpha-désoxy-5-benzylcarbonyloxy-tétracycline,
9-t-butyl-6-alpha-désoxy-5-diméthylaminocarbonyloxy-tétracycline,
9-t-butyl-6-alpha-désoxy-5-cyclopentylcarbonyloxy-tétracycline,
9-t-butyl-6-alpha-désoxy-5-cyclobutylcarbonyloxy-tétracycline,
9-t-butyl-6-alpha-désoxy-5-cyclohexylcarbonyloxy-tétracycline,
9-t-butyl-6-alpha-désoxy-5-cycloheptylcarbonyloxy-tétracycline,
9-[(diméthylamino)-t-butyl]-6-alpha-désoxy-5-formyloxy-tétracycline,
9-[(diméthylamino)-t-butyl]-6-alpha-désoxy-5-acétoxy-tétracycline,
9-[(diméthylamino)-t-butyl]-6-alpha-désoxy-5-propionylcarbonyloxy-tétracycline,
9-[(diméthylamino)-t-butyl]-6-alpha-désoxy-5-phénylcarbonyloxy-tétracycline,
9-[(diméthylamino)-t-butyl]-6-alpha-désoxy-5-benzylcarbonyloxy-tétracycline,
9-[(diméthylamino)-t-butyl]-6-alpha-désoxy-5-diméthylaminocarbonyloxy-tétracycline,
9-[(diméthylamino)-t-butyl]-6-alpha-désoxy-5-cyclopentylcarbonyloxy-tétracycline,
9-[(diméthylamino)-t-butyl]-6-alpha-désoxy-5-cyclobutylcarbonyloxy-tétracycline,
9-[(diméthylamino)-t-butyl]-6-alpha-désoxy-5-cyclohexylcarbonyloxy-tétracycline,
9-[(diméthylamino)-t-butyl]-6-alpha-désoxy-5-cycloheptylcarbonyloxy-tétracycline, et
des sels pharmaceutiquement acceptables de ceux-ci.

3. Le composé de la revendication 1, dans lequel R est un alkyle possédant 1 à 20 atomes de carbone, un alcényle possédant 2 à 20 atomes de carbone, un alcynyle possédant 2 à 20 atomes de carbone, un alkoxy possédant 1 à 20 atomes de carbone, un alkylthio possédant 1 à 20 atomes de carbone, un alkylsulfinyle possédant 1 à 20 atomes de carbone, un alkylsulfonyle possédant 1 à 20 atomes de carbone, un groupe alkyle possédant 1 à 20 atomes de carbone et auquel est lié un groupe aminé, ou un arylalkyle ;
R² est un alkyle possédant 1 à 20 atomes de carbone, un alcényle possédant 2 à 20 atomes de carbone, un alcynyle possédant 2 à 20 atomes de carbone, un alkoxy possédant 1 à 20 atomes de carbone, un alkylthio possédant 1 à 20 atomes de carbone, un alkylsulfinyle possédant 1 à 20 atomes de carbone, un alkylsulfonyle possédant 1 à 20 atomes de carbone, un groupe alkyle possédant 1 à 20 atomes de carbone et auquel est lié un groupe aminé, un arylalkyle, un alcanoyle possédant 1 à 20 atomes de carbone, un aroyle, un alcaroyle, un aryle carbocyclique, ou un hétéroaromatique ; et
Z est un hydrogène, un alkyle possédant 1 à 20 atomes de carbone, un alcényle possédant 2 à 20 atomes de carbone, un alcynyle possédant 2 à 20 atomes de carbone, un alkoxy possédant 1 à 20 atomes de carbone, un alkylthio possédant 1 à 20 atomes de carbone, un alkylsulfinyle possédant 1 à 20 atomes de carbone, un alkylsulfonyle possédant 1 à 20 atomes de carbone, un groupe alkyle possédant 1 à 20 atomes de carbone et auquel est lié un groupe aminé, un arylalkyle, un aryle carbocyclique, ou un groupe hétéroalicyclique.

4. Le composé de la revendication 3, dans lequel Z est un hydrogène ; et R est un alkyle possédant 1 à 12 atomes de carbone, un alcényle possédant 2 à 12 atomes de carbone, un alcynyle possédant 2 à 12 atomes de carbone, un alkoxy possédant 1 à 12 atomes de carbone, un alkylthio possédant 1 à 12 atomes de carbone, un alkylsulfinyle possédant 1 à 12 atomes de carbone, un alkylsulfonyle possédant 1 à 12 atomes de carbone, un groupe alkyle possédant 1 à 12 atomes de carbone et auquel est lié un groupe aminé, ou un benzyle.

5. Le composé de la revendication 1, dans lequel R est un alkyle possédant 1 à 12 atomes de carbone, un alcényle possédant 2 à 12 atomes de carbone, un alcynyle possédant 2 à 12 atomes de carbone, un alkoxy possédant 1 à 12 atomes de carbone, un alkylthio possédant 1 à 12 atomes de carbone, un alkylsulfinyle possédant 1 à 12 atomes de carbone, un alkylsulfonyle possédant 1 à 12 atomes de carbone, un groupe alkyle possédant 1 à 12 atomes de carbone et auquel est lié un groupe aminé, ou un benzyle ;
R² est un alkyle possédant 1 à 12 atomes de carbone, un alcényle possédant 2 à 12 atomes de carbone, un alcynyle possédant 2 à 12 atomes de carbone, un alkoxy possédant 1 à 12 atomes de carbone, un alkylthio possédant 1 à 12 atomes de carbone, un alkylsulfinyle possédant 1 à 12 atomes de carbone, un alkylsulfonyle possédant 1 à 12 atomes de carbone, un groupe alkyle possédant 1 à 12 atomes de carbone et auquel est lié un groupe aminé, un benzyle, un aroyle, un alcaroyle, un aryle carbocyclique, ou un hétéroaromatique ;
et Z est un hydrogène.

6. Le composé de la revendication 1, dans lequel R est choisi dans le groupe consistant en t-butyl, chloro-t-butyl, pipéridinoéthyl, et (diméthylamino)-t-butyl ; et R² est choisi dans le groupe consistant en propionate, formyloxy, acétoxy, propionyloxy, phénylcarbonyloxy, benzylcarbonyloxy, acétylcarbonyloxy, propionylcarbonyloxy, phénylcarbonyloxy, benzylcarbonyloxy, diméthylaminocarbonyloxy, cyclopentylcarbonyloxy, cyclobutylcarbonyloxy, cyclohexylcarbonyloxy, et cycloheptylcarbonyloxy ; et Z est un hydrogène.

7. Le composé de la revendication 1, dans lequel ledit composé est choisi dans le groupe consistant en :
9-t-butyl-6-désoxy-5-propionylcarbonyloxytétracycline,
9-t-butyl-6-désoxy-5-acétylcarbonyloxytétracycline,
9-t-butyl-6-désoxy-5-cyclobutylcarbonyloxytétracycline, et
des sels pharmaceutiquement acceptables de ceux-ci.

8. Composé de formule II suivante : 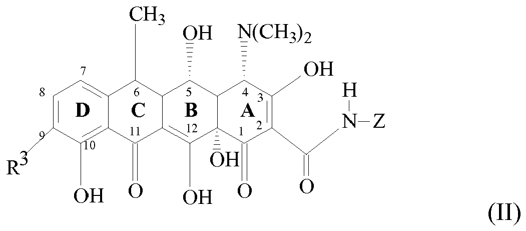
dans lequel R³ est un alkyle, un alcényle, un alcynyle, un alkoxy, un alkylthio, un alkylsulfinyle, un alkylsulfonyle, un alkylaryle ;
Z est un hydrogène, un alkyle, un alcényle, un alcynyle, un alkoxy, un alkylthio, un alkylsulfinyle, un alkylsulfonyle, un arylalkyle, un aryle carbocyclique, un hétéroalicyclique, un groupe alkyle auquel est lié un groupe aminé, ou un groupe hétéroaromatique ;
ou un sel pharmaceutiquement acceptable de ceux-ci.

9. Le composé de la revendication 8, dans lequel R³ est un alkyle possédant 1 à 20 atomes de carbone, un alcényle possédant 2 à 20 atomes de carbone, un alcynyle possédant 2 à 20 atomes de carbone, un alkoxy possédant 1 à 20 atomes de carbone, un alkylthio possédant 1 à 20 atomes de carbone, un alkylsulfinyle possédant 1 à 20 atomes de carbone, un alkylsulfonyle possédant 1 à 20 atomes de carbone, un groupe alkyle possédant 1 à 20 atomes de carbone et auquel est lié un groupe aminé, ou un arylalkyle ; et
Z est un hydrogène, un alkyle possédant 1 à 20 atomes de carbone, un alcényle possédant 2 à 20 atomes de carbone, un alcynyle possédant 2 à 20 atomes de carbone, un alkoxy possédant 1 à 20 atomes de carbone, un alkylthio possédant 1 à 20 atomes de carbone, un alkylsulfinyle possédant 1 à 20 atomes de carbone, un alkylsulfonyle possédant 1 à 20 atomes de carbone, un groupe alkyle possédant 1 à 20 atomes de carbone et auquel est lié un groupe aminé, un arylalkyle, un aryle carbocyclique, ou un groupe hétéroalicyclique.

10. Le composé de la revendication 8, dans lequel ledit composé est choisi dans le groupe consistant en :
9-t-butyl-6-désoxy-5-hydroxytétracycline,
9-[1'-(1'-méthyl) cyclohexyl]-6-désoxy-5-hydroxytétracycline,
9-[1'-(1'-méthyl) cyclopentyl]-6-désoxy-5-hydroxytétracycline,
9-[1'-(1'-méthyl) cyclobutyl]-6-désoxy-5-hydroxytétracycline,
9-[2'-(2'-méthyl)pentyl]-6-désoxy-5-hydroxytétracycline,
9-[4'-(1'-bromo-4'-méthyl) pentyl]-6-désoxy-5-hydroxytétracycline,
9-[4'-(1'-diméthylamino-4'-méthyl) pentyl]-6-désoxy-5-hydroxytétracycline,
9-[4'-(1'-pyrrolidinyl-4'-méthyl) pentyl]-6-désoxy-5-hydroxytétracycline,
9-[4'-(1'-cyano-4'-méthyl) pentyl]-6-désoxy-5-hydroxytétracycline,
9-[4'-(1'-nitro-4'-méthyl) pentyl]-6-désoxy-5-hydroxytétracycline,
9-[4'-(1'-acétoxy-4'-méthyl) pentyl]-6-désoxy-5-hydroxytétracycline,
9-(chloro-t-butyl)-6-désoxy-oxytétracycline,
9-[(diméthylamino)-t-butyl]-6-désoxy-oxytétracycline,
9-(amino)-t-butyl-6-désoxy-oxytétracycline,
9-[(pipéridino)-t-butyl]-6-désoxy-oxytétracycline,
9-[(diéthylamino)-t-butyl]-6-désoxy-oxytétracycline,
9-[(dipropylamino)-t-butyl]-6-désoxy-oxytétracycline,
et des sels pharmaceutiquement acceptables de ceux-ci.

11. Le composé de la revendication 9, dans lequel R³ est un alkyle possédant 1 à 12 atomes de carbone, un alcényle possédant 2 à 12 atomes de carbone, un alcynyle possédant 2 à 12 atomes de carbone, un alkoxy possédant 1 à 12 atomes de carbone, un alkylthio possédant 1 à 12 atomes de carbone, un alkylsulfinyle possédant 1 à 12 atomes de carbone, un alkylsulfonyle possédant 1 à 12 atomes de carbone, un groupe alkyle possédant 1 à 12 atomes de carbone et auquel est lié un groupe aminé, ou un benzyle ; et
Z est un hydrogène.

12. Le composé de la revendication 9, dans lequel R³ est choisi dans le groupe consistant en t-butyl, chloro-t-butyl, (diméthylamino)-t-butyl, méthylcyclohexyl, méthylcyclobutyl, méthylpentyl, bromométhylpentyl, nitrométhylpentyl, et acétoxyméthylpentyl.

13. Composition pharmaceutique comprenant un composé tel que défini dans l'une quelconque des revendications 1 à 12.

14. Composé de l'une quelconque des revendications 1 à 12 destiné à être utilisé comme médicament.

15. Composé de l'une quelconque des revendications 1 à 12 destiné à être utilisé dans un traitement contre un micro-organisme ciblé.

16. Composé de l'une quelconque des revendications 1 à 12 destiné à être utilisé dans un traitement contre une bactérie.

17. Méthode de transformation de bactéries résistantes à la tétracycline en bactéries sensibles à la tétracycline, comprenant
a) mise en contact des bactéries résistantes avec une quantité prédéterminée d'un composé de l'une quelconque des revendications 1 à 12, et
b) administration concomitante aux bactéries d'une quantité prédéterminée d'un composé de type tétracycline qui est différent du composé de l'étape a).
